Europäisches Patentamt

European Patent Office

Office européen des brevets

(19)

(11)     **EP 1 700 606 A1**

## (12)     EUROPEAN PATENT APPLICATION

(43) Date of publication:
    **13.09.2006 Bulletin 2006/37**

(21) Application number: **06009570.0**

(22) Date of filing: **14.09.2000**

(51) Int Cl.:
    *A61K 39/395* [(2006.01)]     *A61P 13/12* [(2006.01)]
    *A61K 38/17* [(2006.01)]     *C12N 15/13* [(2006.01)]

| | |
|---|---|
| (84) Designated Contracting States:<br>**AT BE CH CY DE DK ES FI FR GB GR IE IT LI LU MC NL PT SE**<br>Designated Extension States:<br>**AL LT LV MK RO SI** | • **IMPERIAL COLLEGE OF SCIENCE, TECHNOLOGY AND MEDICINE**<br>**London SW7 2AZ (GB)** |
| (30) Priority: **14.09.1999 US 153826 P** | (72) Inventor: **The designation of the inventor has not yet been filed** |
| (62) Document number(s) of the earlier application(s) in accordance with Art. 76 EPC:<br>**00961900.8 / 1 214 091** | (74) Representative: **Vossius & Partner**<br>**Siebertstrasse 4**<br>**81675 München (DE)** |
| (71) Applicants:<br>• **Biogen Idec MA, Inc.**<br>**Cambridge**<br>**Massachusetts 02142 (US)** | Remarks:<br>This application was filed on 09.05.2006 as a divisional application to the application mentioned under INID code 62. |

(54)     **Therapies for chronic renal failure using one or more integrin antagonists**

(57)     The present invention provides methods for the treatment, and pharmaceuticals for use in the treatment, of mammalian subjects in, or at risk of chronic renal failure, or at risk of a need for renal replacement therapy. The methods involve the administration of certain integrin antagonists.

**EP 1 700 606 A1**

**Description**

Field of the Invention

The present invention relates generally to methods of treatment for renal disease. In particular; the invention relates to methods of treatment for conditions which place mammals, including humans, in, or at risk of, chronic renal failure. The methods involve the administration of certain integrin antagonists.

Background of the Invention

Many physiological processes require that cells come into close contact with other cells and/or extracellular matrix. Such adhesion events may be required for cell activation, migration (e.g., leukocyte migration), proliferation and differentiation. Cell-cell and cell-matrix interactions are mediated through several families of cell adhesion molecules, one family of which includes the integrins.

Cell adhesion molecules play an essential role in both normal and pathophysiological processes. Therefore, the targeting of specific and relevant molecules in certain disease conditions without interfering with normal cellular functions is essential for an effective and safe therapeutic agent that inhibits cell-cell and cell-matrix interactions.

For example, leukocyte migration into glomeruli is a typical feature of human glomerulonephritis (GN) and leukocytes are key mediators of kidney damage. A similar leukocyte migration is seen in animal models of GN. See Allen et al., Journal of Immunology, 162: 5519-5527 (1999) and references cited therein. We have previously demonstrated that blocking the integrin VLA-4 inhibits acute nephrotoxic nephritis in a rat model of this disease (Allen et al., supra; Tam, F.W.K. et al., Nephrol. Dial. Transplant. 14: 1658-1666 (1999).

Although there is some information on the role of integrins in acute renal disease, there is nevertheless no current information available that clarifies the importance of cell adhesion molecules in chronic renal disease. One cannot assume, *a priori,* that the importance of a cell adhesion molecule in acute renal disease will necessarily mean that such molecules are important in chronic renal disease (See Examples).

Chronic renal failure (CRF) may be defined as a progressive, permanent and significant reduction of glomerular filtration rate (GFR) due to a significant and continuing loss of nephrons. Chronic renal failure typically begins from a point at which a chronic renal insufficiency (i.e., a permanent decrease in renal function of at least 50-60%) has resulted from some insult to the renal tissues which has caused a significant loss of nephron units. The initial insult may or may not have been associated with an episode of acute renal failure or it may be associated with any number of renal disorders including, but not limited to, end-stage renal disease, chronic diabetic nephropathy, diabetic glomerulopathy, diabetic renal hypertrophy, hypertensive nephrosclerosis, hypertensive glomerulosclerosis, chronic glomerulonephritis, hereditary nephritis, renal dysplasia and chronic rejection following renal allograft transplantation. Irrespective of the nature of the initial insult, chronic renal failure manifests a "final common path" of signs and symptoms as nephrons are progressively lost and GFR progressively declines. This progressive deterioration in renal function is slow, typically spanning many years or decades in human patients, but seemingly inevitable.

In humans, as chronic renal failure progresses, and GFR continues to decline to less than 10% of normal (e.g., 5-10 ml/min), the subject enters end-stage renal disease (ESRD). During this phase, the inability of the remaining nephrons to adequately remove waste products from the blood, while retaining useful products and maintaining fluid and electrolyte balance, leads to a decline in which many organ systems, and particularly the cardiovascular system, may rapidly begin to fail. At this point, renal failure will rapidly progress to death unless the subject receives renal replacement therapy (i.e., chronic hemodialysis, continuous peritoneal dialysis, or kidney transplantation).

Approximately 600 patients per million receive chronic dialysis each year in the United States, at an average cost approaching $60,000-$80,000 per patient per year. Of the new cases of end-stage renal disease each year, approximately 28-33% are due to diabetic nephropathy (or diabetic glomerulopathy or diabetic renal hypertrophy), 24-29% are due to hypertensive nephrosclerosis (or hypertensive glomerulosclerosis), and 15-22% are due to glomerulonephritis. The 5-year survival rate for all chronic dialysis patients is approximately 40%, but for patients over 65, the rate drops to approximately 20%. A need exists, therefore, for treatments which will prevent the progressive loss of renal function which has caused almost two hundred thousand patients in the United States alone to become dependent upon chronic dialysis, and which results in the premature deaths of tens of thousands each year

Summary of the Invention

We have found that that cell adhesion molecules such as integrins are not only important in the etiology of acute renal disease but are also important in the etiology of CRF. The present invention is directed to methods of treatment, and pharmaceutical preparations for use in the treatment, of mammalian subjects in, or at risk of, chronic renal failure, or at risk of the need for renal replacement therapy. Such subjects include subjects already afflicted with chronic renal failure, or which have already received renal replacement therapy, as well as any subject reasonably expected to suffer a progressive loss of renal function associated with progressive loss of functioning nephron units.

The methods and compositions of this invention capitalize in part upon the discovery that certain agents that are renal therapeutic agents, defined herein as integrin antagonists, including anti- VLA-4 and VLA- 1 antibodies may be used in the treatment of subjects at risk, as defined herein, of chronic renal failure or the need for renal replacement therapy.

The renal therapeutic agents of the invention may be administered by any route of administration which is compatible with the selected agent, and may be formulated with any pharmaceutically acceptable carrier appropriate to the route of administration. Preferred routes of administration are parenteral and, in particular, are subcutaneous, intramuscular, intravenous, intraperitoneal, and renal intracapsular. Daily dosages of the renal therapeutic agents are expected to be in the range of about 0.1 - 50 mg/kg body weight, and more preferably about 1-3 mg/kg body weight, although precise dosages will vary depending upon the particular renal therapeutic agent employed and the particular subject's medical condition and history. Dosages may be given once or several times a week at intervals of 1, 2 or 4 weeks The treatments of the present invention are useful in preventing, inhibiting or delaying the progressive loss of functional nephron units, and the consequent progressive loss of renal function, which typify chronic renal failure. As such, they are of great value in preventing or delaying the need for chronic dialysis or renal replacement therapy in subjects with chronic rena! insufficiency, or reducing the necessary frequency of chronic renal dialysis in subjects with endstage renal disease.

Brief Description of the Figures

Figure 1 shows the measurements of serum creatinine during the chronic renal failure phase of the animal model for rats receiving a soluble TGF beta antagonist (solid square) versus rats receiving the negative control.

Figure 2 is a graph showing mean % glomerular area fibrosed (estimates for 50 consecutive glomeruli) and number of glomerular quadrants occupied by fibrin during the chronic renal failure phase of the animal model for rats receiving a soluble TGF beta antagonist (solid square) versus rats receiving the negative control.

Figure 3 shows the survival curves in the chronic renal failure phase of the animal model for rats receiving negative control and rats receiving anti-VLA- 1 antibody.

Figure 4 shows the survival curves in the chronic renal failure phase of the animal model for rats receiving anti-VLA-4 antibody (closed squares) and rats receiving negative control (open circles).

Detailed Description of the Invention

1. Definitions

In order to more clearly and concisely point out the subject matter of the claimed invention, the following definitions are provided for specific terms used in the following written description and appended claims.

The integrin very late antigen (VLA) superfamily is made up of structurally and functionally related glycoproteins consisting of (alpha and beta) heterodimeric, transmembrane receptor molecules found in various combinations on nearly every mammalian cell type. The integrin superfamily is made up of structurally and functionally related glycoproteins consisting of alpha and beta heterodimeric, transmembrane receptor molecules found in various combinations on nearly every mammalian cell type. For reviews see: E. C. Butcher, Cell, 67, 1033 (1991); D. Cox et al., "The Pharmacology of the Integrins." Medicinal Research Rev. Vol. 195 (1994) and V. W. Engleman et al., "Cell Adhesion Integrins as Pharmaceutical Targets" in Ann. Rev. Medicinal Chemistry, Vol. 31, J.A. Bristol, Ed.; Acad. Press, NY, 1996, p. 191. Integrins of the VLA family include VLA- 1, -2, -3, -4, -5, -6, in which each of the molecules comprise a betal chain non-covalently bound to an alpha chain, (alpha1, alpha2, alpha3, alpha4, alpha5, alpha6), respectively.

In particular, alpha1- beta1 integrin is a cell-surface receptor for collagen I, collagen IV, laminin and possibly other ligands (the latter terms individually and collectively referred to as "alpha1 integrin ligands" or "VLA- 1 ligand(s)" although the term "collagen" will be used throughout to include all such alpha 1 integrin ligands, it being appreciated by workers of ordinary skill in the art that other ligands for alpha1 integrins exist and can be analyzed using conventional methods. The integrin alpha1 beta 1 supports not only collagen-dependent adhesion and migration, but also is likely to be a critical collagen receptor on mesenchymally-derived cells that may be involved in ECM remodeling after injury (Gotwals et al. J. Clin. Invest. 97: 2469- 2477 (1996)). The ability of cells to contract and organize collagen matrices is a critical component of any wound healing response. The term "albl integrin" (or "VLA- 1" or "albl" or "albl integrin", used interchangeably) herein refers to polypeptides or homologs or fragments thereof which are capable of binding to members of the extracellular matrix proteins such as laminin or collagen, or homologs or fragments of such extracellular matrix proteins.

Alpha 4 beta 1 integrin is a cell-surface receptor for VCAM-1, fibronectin and possibly other ligands (the these ligands individually and collectively referred to as "alpha4 ligand(s)"). The term "a4b1 integrin" (or "VLA-4" or "a4b1 " or "a4blintegrin", used interchangeably) herein thus refers to polypeptides or homologs or fragments thereof which are capable of binding to VCAM- 1 and members of the extracellular matrix proteins, most particularly fibronectin, or homologs or fragments of such extracellular matrix proteins, although it will be appreciated by workers of ordinary skill in the art that other ligands for VLA-4 may exist and can be analyzed using conventional methods. Nevertheless, it is known that the alpha4 subunit will associate with other beta subunits besides betal so that we may define the term "alpha 4 integrin" as being those integrins whose alpha4 subunit associates with one or another of the beta subunits. An example of an "alpha4" integrin is alpha4beta7 (R. Lobb and M. Hemler, J. Clin: Invest., 94: 1722-1728 (1994). As used herein, the term "integrin(s)" means VLA-1 and/or VLA-4, as well as integrins that contain beta 1, beta7 or any other beta subunit.

An integrin "antagonist" includes any compound that inhibits integrins from binding with an integrin ligand and/or receptor. Anti-integrin antibody or antibody homolog-containing proteins (discussed below) as well as other molecules such as soluble forms of the ligand proteins for integrins are useful. Soluble forms of the ligand proteins for integrins include soluble VCAM- 1 or collagen peptides, VCAM- 1 fusion proteins, or bifunctional VCAM-1 Ig fusion proteins. For example, a soluble form of an integrin ligand or a fragment thereof may be administered to bind to integrin, and preferably compete for an integrin binding site on cells, thereby leading to effects similar to the administration of antagonists such as anti-integrin (e.g., VLA-II and/or VLA-4) antibodies. In particular, soluble integrin mutants that bind ligand but do not elicit integrin-dependent signaling are included within the scope of the invention. Such integriri mutants can act as competitive inhibitors of wild type integrin protein and are considered "antagonists". Other antagonists used in the methods of the invention are "small molecules", as defined below. Also included within the invention are methods using molecules that antagonize the action of more than one integrin, such as small molecules or antibody homologs that antagonize both alpha 4 integrins (e.g., VLA-4) and VLA-1, or other combinations of integrins. Also included within the scope of the invention are methods using a combination of molecules such that the combination antagonizes the action of more than one integrin, such as methods using several small molecules or antibody homologs that in combination antagonize both alpha 4 integrins (e.g., VLA-4) and VLA-1, or other combinations of integrins.

As discussed herein, certain integrin antagonists can be fused or otherwise conjugated to, for instance, an antibody homolog such as an immunoglobulin or fragment thereof and are not limited to a particular type or structure of an integrin or ligand or other molecule. Thus, for purposes of the invention, any agent capable of forming a fusion protein (as defined below) and capable of binding to integrin ligands and which effectively blocks or coats VLA- 1 and/or alpha 4 (e.g., VLA-4) integrin is considered to be an equivalent of the antagonists used in the examples herein.

"Antibody homolog" includes intact antibodies consisting of immunoglobulin light and heavy chains linked via disulfide bonds. The term "antibody homolog" is also intended to encompass a protein comprising one or more polypeptides selected from immunoglobulin light chains, immunoglobulin heavy chains and antigen-binding fragments thereof which are capable of binding to one or more antigens (i.e., integrin or integrin ligand). The component polypeptides of an antibody homolog composed of more than one polypeptide may optionally be disulfide-bound or otherwise covalently crosslinked. Accordingly, therefore, "antibody homologs" include intact immunoglobulins of types IgA, IgG, IgE, IgD, IgM (as well as subtypes thereof), wherein the light chains of the immunoglobulin may be of types kappa or lambda. "Antibody homologs" also includes portions of intact antibodies that retain antigen binding specificity, for example Fab fragments, Fab' fragments, F(ab')2 fragments, F(v) fragments, heavy and light chain monomers or dimers or mixtures thereof.

"Humanized antibody homolog" is an antibody homolog, produced by recombinant DNA technology, in which some or all of the amino acids of a human immunoglobulin light or heavy chain that are not required for antigen binding have been substituted for the corresponding amino acids from a nonhuman mammalian immunoglobulin light or heavy chain.

As used herein, a "human antibody homolog" is an antibody homolog produced by recombinant DNA technology, in which all of the amino acids of an immunoglobulin light or heavy chain that are derived from a human source (regardless of whether or not such amino acids are required for antigen binding).

An integrin "agonist" includes any compound that activates the integrin ligand.

"Amino acid" is a monomeric unit of a peptide, polypeptide, or protein. There are twenty amino acids found in naturally occurring peptides, polypeptides and proteins, all of which are L-isomers. The term also includes analogs of the amino acids and D-isomers of the protein amino acids and their analogs.

The term "antagonist biological activity" means that the antagonists of the invention or functional equivalents or variants thereof; are capable of significantly preventing, inhibiting, delaying or alleviating the progressive loss of renal function in a standard animal model of chronic renal failure, or are capable of causing a clinically significant improvement in a standard marker of renal function when administered to a mammal in, or at risk of, chronic renal failure.

"Broad cast" refers to the formed elements which may be present in urine that rare cylindrical masses of agglutinated materials that typically represent a mold or "cast" of the lumen of a distal convoluted tubule or Collecting tubule. In CRF subjects, however, hypertrophy of the tubules may result in the presence of "broad casts" or "renal failure casts" which are 2-6 times the diameter of normal casts and often have a homogeneous waxy appearance. Microscopic examination of urinary sediment for the presence of formed elements is a standard procedure in urinalysis.

"Bioavailability" refers to the ability of a compound to be absorbed by the body after administration. For instance, a first compound has greater bioavailability than a second compound if, when both are administered in equal amounts, the first compound is absorbed into the blood to a greater extent than the second compound.

"Chronic" means persisting for a period of at least three, and more preferably, at least six months. Thus, for example, a subject with a measured GFR chronically below 50% of the expected GFR is a subject in which the GFR has been measured and found to be below 50% of the expected GFR in at least two measurements separated by at least three, and more preferably, by at least six months, and for which there is no medically sound reason to believe that GFR was substantially (e.g., 10%) higher during the intervening period. Those having ordinary skill in the art will understand that, with respect to animal models, the term "chronic" may mean differing time periods that depend upon the type of animal model.

"Covalently coupled" means that the specified moieties of the invention (e.g., immunoglobulin fragment/integrin antagonist) are either directly covalently bonded to one another, or else are indirectly covalently joined to one another through an intervening moiety or moieties, such as a bridge, spacer, or linkage moiety or moieties. The intervening moiety or moieties are called a "coupling group". The term "conjugated" is used interchangeably with "covalently coupled".

"Expression control sequence" is a sequence of polynucleotides that controls and regulates expression of genes when operatively linked to those genes.

"Expression vector" is a polynucleotide, such as a DNA plasmid or phage (among other common examples) which allows expression of at least one gene when the expression vector is introduced into a host cell. The vector may, or may not, be able to replicate in a cell.

The phrase "extracellular signaling protein" means any protein that is either secreted from a cell, or is associated with the cell membrane, and upon binding to the receptor for that protein on a target cell, triggers a response in the target cell.

An "effective amount" of an agent of the invention is that amount which produces a result or exerts an influence on the particular condition being treated.

"Functional equivalent" of an amino acid residue is (i) an amino acid having similar reactive properties as the amino acid residue that was replaced by the functional equivalent; (ii) an amino acid of an antagonist of the invention, the amino acid having similar properties as the amino acid residue that was replaced by the functional equivalent; (iii) a non-amino acid molecule having similar properties as the amino acid residue that was replaced by the functional equivalent. A first polynucleotide encoding a proteinaceous antagonist of the invention is "functionally equivalent" compared with a second polynucleotide encoding the antagonist protein if it satisfies at least one of the following conditions: 1. the "functional equivalent" is a first polynucleotide that hybridizes to the second polynucleotide under standard hybridization conditions and/or is degenerate to the first polynucleotide, sequence. Most preferably, it encodes a mutant protein having the activity of an integrin antagonist protein; 2. The "functional equivalent" is a first polynucleotide that codes on expression for an amino acid sequence encoded by the second polynucleotide.

The integrin antagonists used in the invention include, but are not limited to, the agents listed herein as well as their functional equivalents. As used herein, the term "functional equivalent" therefore refers to an integrin antagonist or a polynucleotide encoding the integrin antagonist that has the same or an improved beneficial effect on the recipient as the integrin antagonist of which it is deemed a functional equivalent. As will be appreciated by one of ordinary skill in the art, a functionally equivalent protein can be produced by recombinant techniques, e.g., by expressing a "functionally equivalent DNA". Accordingly, the instant invention embraces integrin proteins encoded by naturally-occurring DNAS, as well as by nonnaturally-occurring DNAs which encode the same protein as encoded by the naturally-occurring DNA. Due to the degeneracy of the nucleotide coding sequences, other polynucleotides may be used to encode integrin protein. These include all, or portions of the above sequences which are altered by the substitution of different codons that encode the same amino acid residue within the sequence, thus producing a silent change. Such altered sequences are regarded as equivalents of these sequences. For example, Phe (F) is coded for by two codons, TTC or TTT, Tyr (Y) is coded for by TAC or TAT and His (H) is coded for by CAC or CAT. On the other hand, Trp (W) is coded for by a single codon, TGG. Accordingly, it will be appreciated that for a given DNA sequence encoding a particular integrin there will be many DNA degenerate sequences that will code for it. These degenerate DNA sequences are considered within the scope of this invention.

The term "fusion" or "fusion protein" refers to a co-linear, covalent linkage of two or more proteins or fragments thereof via their individual peptide backbones, most preferably through genetic expression of a polynucleotide molecule encoding those proteins. It is preferred that the proteins or fragments thereof are from different sources so that this type of fusion protein is called a "chimeric" molecule. Thus, preferred fusion proteins are chimeric proteins that include an integrin antagonist or fragment covalently linked to a second moiety that is not a integrin antagonist. Preferred fusion proteins of the invention may include portions of intact antibodies that retain antigen-binding specificity, for example, Fab fragments, Fab' fragments; F(ab')2 fragments, F(v) fragments, heavy chiin monomers or dimers, light chain monomers or dimers, dimers consisting of one heavy and one light chain; and the like.

The most preferred fusion proteins are chimeric and comprise an integrin antagonist moiety fused or otherwise linked to all or part of the hinge and constant regions of an immunoglobulin light chain, heavy chain, or both. Thus, this invention features a molecule which includes: (1) a integrin antagonist moiety, (2) a second peptide, e.g., one which increases solubility or in vivo life time of the integrin antagonist moiety, e.g., a member of the immunoglobulin super family or fragment or portion thereof, e.g., a portion or a fragment of IgG, e.g., the human IgG1 heavy chain constant region, e.g., CH2, CH3, and hinge regions. Specifically, a "integrin antagonist/Ig fusion" is a protein comprising a biologically active integrin antagonist molecule of the invention (e.g. a soluble VLA-4 ligand, or a biologically active fragment thereof) linked to an N-terminus of an immunoglobulin chain wherein a portion of the N-terminus of the immunoglobulin is replaced with the integrin antagonist. A species of integrin antagonist/Ig fusion is an "integrin /Fc fusion" which is a protein comprising an integrin antagonist of the invention linked to at least a part of the constant domain of an immunoglobulin. A preferred Fe fusion comprises a integrin antagonist of the invention linked to a fragment of an antibody containing the C terminal domain of the heavy immunoglobulin chains.

The term "fusion protein" also means an integrin antagonist chemically linked via a mono- or hetero- functional molecule to a second moiety that is not an integrin antagonist (resulting in a "chimeric" molecule) and is made de novo from purified protein as described below. Thus, one example of a chemically linked, as opposed to recombinantly linked, chimeric molecule that is a fusion protein may comprise: (1) an alpha 4 integrin subunit targeting moiety, (e.g., a VCAM-1 moiety capable of binding to VLA-4) on the surface of VLA-4 bearing cells; (2) a second molecule which increases solubility or in vivo life time of the targeting moiety, (e.g., a polyalkylene glycol polymer such as polyethylene glycol (PEG)). The alpha4 targeting moiety can be any naturally occurring alpha4 ligand or fragment thereof, e.g., a VCAM- 1 peptide or a similar conservatively substituted amino acid sequence.

"Heterologous promoter" as used herein is a promoter which is not naturally associated with gene or a purified nucleic acid.

"Homology" as used herein is synonymous with the term "identity" and refers to the sequence similarity between two polypeptides, molecules, or between two nucleic acids. When a position in both of the two compared sequences is occupied by the same base or amino acid monomer subunit (for instance, if a position in each of the two DNA molecules is occupied by adenine, or a position in each of two polypeptides is occupied by a lysine), then the respective molecules are homologous at that position. The percentage homology between two sequences is a function of the number of matching or homologous positions shared by the two sequences divided by the number of positions compared x 100. For instance, if 6 of 10 of the positions in two sequences are matched or are homologous, then the two sequences are 60% homologous. By way of example, the DNA sequences CTGACT and CAGGTT share 50% homology (3 of the 6 total positions are matched). Generally, a comparison is made when two sequences are aligned to give maximum homology. Such alignment can be provided using, for instance, the method of Karlin and Altschul described in more detail below.

Homologous sequences share identical or similar amino acid residues, where similar residues are conservative substitutions for, or "allowed point mutations" of, corresponding amino acid residues in an aligned reference sequence. In this regard, a "conservative substitution" of a residue in a reference sequence are those substitutions that are physically or functionally similar to the corresponding reference residues, e.g., that have a similar size, shape, electric charge, chemical properties, including the ability to form covalent or hydrogen bonds, or the like. Particularly preferred conservative substitutions are those fulfilling the criteria defined for an "accepted point mutation" in Dayhoff et al., 5: Atlas of Protein Sequence and Structure, 5: Suppl. 3, chapter 22: 354-352, Nat. Biomed. Res. Foundation, Washington, D.C. (1978). "Homology" and "identity" are interchangeable herein and each refer to sequence similarity between two polypeptide sequences. Homology and identity can be determined by comparing a position in each sequence which may be aligned for purposes of comparison. When a position in the compared sequence is occupied by the same amino acid residue, then the polypeptides can be referred to as identical at that position; when the equivalent site is occupied by the same amino acid (e.g., identical) or a similar amino acid (e.g., similar in steric and/or electronic nature), then the molecules can be refered to as homologous at that position. A percentage of homology or identity between sequences is a function of the number of matching or homologous positions shared by the sequences. An "unrelated" or "non-homologous" sequence shares less than 40 percent identity, though preferably less than 25 percent identity, with a sequence of the present invention.

"Percent homology" of two amino acids sequences or two nucleic acid sequences is determined using the alignment algorithm of Karlin and Altschul (Proc. Nat. Acad. Sci., USA 87: 2264 (1990) as modified in Karlin and Altschul (Proc. Nat. Acad. Sci., USA 90: 5873 (1993). Such an algorithm is incorporated into the NBLAST or XBLAST programs of Altschul et al., J. Mol. Biol.. 215:.403 (1990). BLAST searches are performed with the NBLAST program, score = 100, wordlength = 12, to obtain nucleotide sequences homologous to a nucleic acid of the invention. BLAST protein searches are performed with the XBLAST program, score = 50, wordlength = 3, to obtain amino acid sequences homologous to a reference polypeptide. To obtain gapped alignments for comparisons, gapped BLAST is used as described in Altschul et a]., Nucleic Acids Res., 25: 3389 (1997). When using BLAST and Gapped BLAST, the default parameters of the respective programs (XBLAST and NBLAST) are used. See http://www/ncbi.nlm.nih.gov.

"Isolated" (used interchangeably with "substantially pure")- when applied to nucleic acid i.e., polynucleotide sequences that encode integrin antagonists, means an RNA or DNA polynucleotide, portion of genomic polynucleotide, cDNA or synthetic polynucleotide which, by virtue of its origin or manipulation: (i) is not associated with all of a polynucleotide with which it is associated in nature (e.g., is present in a host cell as an expression vector,. or a portion thereof); or (ii) is linked to a nucleic acid or other chemical moiety other than that to which it is linked in nature; or (iii) does not occur in nature. By "Isolated" it is further meant a polynucleotide sequence that is: (i) amplified *in vitro* by, for example, polymerase chain reaction (PCR); (ii) synthesized chemically; (iii) produced recombinantly by cloning; or (iv) purified, as by cleavage and gel separation.

"Isolated" (used interchangeably with "substantially pure") when applied to polypeptides "means a polypeptide or a portion thereof which, by virtue of its origin or manipulation: (i) is present in a host cell as the expression product of a portion of an expression vector; or (ii) is linked to a protein or other chemical moiety other than that to which it is linked in nature; or (iii) does not occur in nature, for example, a protein that is chemically manipulated by appending, or adding at least one hydrophobic moiety to the protein so that the protein is in a form not found in nature.. By "isolated" it is further meant a protein that is : (i) synthesized chemically; or (ii) expressed in a host cell and purified away from associated and contaminating proteins. The term generally means a polypeptide that has been separated from other proteins and nucleic acids with which it naturally occurs. Preferably, the polypeptide is also separated from substances such as antibodies or gel matrices (polyacrylamide) which are used to purify it.

"Multivalent protein complex" refers to a plurality of integrin antagonists (i.e., one or more). For instance, an anti-integrin antibody homolog or fragment may be cross-linked or bound to another antibody homolog or fragment. Each protein may be the same or different and each antibody homolog or fragment may be the same or different.

"Mutant" is any change in the genetic material of an organism, in particular any change (i.e., deletion, substitution, addition, or alteration) in a wild type polynucleotide sequence or any change in a wild type protein. The term "mutein" is used interchangeably with "mutant".

"Operatively linked"- a polynucleotide sequence (DNA, RNA) is operatively linked to expression control sequence when the expression control sequence controls and regulates the transcription and translation of that polynucleotide sequence. The term "operatively linked" includes having an appropriate start signal (e.g., ATG) in front of the polynucleotide sequence to be expressed, and maintaining the correct reading frame to permit expression of the polynucleotide sequence under the control of the expression control sequence, and production of the desired polypeptide encoded by the polynucleotide sequence.

"Protein" is any polymer consisting essentially of any of the 20 amino acids. Although "polypeptide" is often used in reference to relatively large polypeptides, and "peptide" is often used in reference to small polypeptides, usage of these terms in the art overlaps and is varied. The term "protein" as used herein refers to peptides, proteins and polypeptides, unless otherwise noted. The terms "peptide(s)", "protein(s)" and "polypeptide(s)" are used interchangeably herein.

The terms "polynucleotide sequence" and "nucleotide sequence" are also used interchangeably herein.

"Recombinant," as used herein, means that a protein is derived from recombinant, expression systems. Since integrins are not glycosylated nor contain disulfide bonds, they can be expressed in most prokaryotic and eukaryotic expression systems.

"Small molecule"has the definition as in Section ILA2.

"Spacer" sequence refers to a moiety that may be inserted between an amino acid to be modified with an antibody homolog or fragment and the remainder of the protein. A spacer is designed to provide separation between the modification and the rest of the protein so as to prevent the modification from interfering with protein function and/or make it easier for the modification to link with an antibody homolog moiety or any other moiety.

A "substantially pure nucleic acid" is a nucleic acid which is not immediately contiguous with one or both of the coding sequences with which it is normally contiguous in the naturally occurring genome of the organism from which the nucleic acid is derived. Substantially pure DNA also includes a recombinant DNA which is part of a hybrid gene encoding additional integrin sequences.

The phrase "surface amino acid" means any amino acid that is exposed to solvent when a protein is folded in its native form.

"Hybridization conditions" generally mean salt and temperature conditions substantially equivalent to 0.5 X SSC to about 5 X SSC and 65 ° C for both hybridization and wash. The term "standard hybridization conditions" as used herein is therefore an operational definition and encompasses a range of hybridization conditions. Nevertheless, "high stringency" conditions include hybridizing with plaque screen buffer (0.2% polyvinylpyrrolidone, 0.2% Ficoll 400; 0.2% bovine serum albumin, 50 mM Tris-HCI (pH 7.5); 1 M NaCl; 0.1% sodium pyrophosphate; 1 % SDS); 10% dextran sulfate, and 100 $\mu$ g/ml denatured, sonicated salmon sperm DNA at 65 ° C for 12-20 hours, and washing with 75 mM NaCl/7.5 mM sodium citrate (0.5 x SSC)/1% SDS at 65° C. "Low stringency" conditions include hybridizing with plaque screen buffer, 10% dextran sulfate and 116 g g/ml denatured, sonicated salmon sperm DNA at 55 ° C for 12-20 hours, and washing with 300 mM NaCl/30mM sodium citrate (2.0 X SSC)/1% SDS at 55 ° C. See also Current Protocols in Molecular Biology, John Wiley & Sons, Inc. New York, Sections 6.3.1-6.3.6, (1989).

A "subject in, or at risk of, chronic renal failure" may also be at risk of the need for renal replacement therapy (i.e., chronic hemodialysis, continuous peritoneal dialysis, or kidney transplantation), if the subject is a human patient and is reasonably expected to suffer a progressive loss of renal function associated with progressive loss of functioning nephron units. Whether a particular subject in a particular animal model or clinical trial is in, or at risk of, chronic renal failure is a determination which may routinely be made by one of ordinary skill in the relevant medical or veterinary art. Subjects in, or at risk of, chronic renal failure (or at risk of the need for renal replacement therapy) include, but are not limited to, the following: subjects which may be regarded as afflicted with chronic renal, failure, end-stage renal disease, chronic diabetic nephropathy, hypertensive nephrosclerosis, chronic glomerulonephritis, hereditary nephritis, and/or renal dysplasia; subjects having a biopsy indicating glomerular hypertrophy, tubular hypertrophy, chronic glomerulosclerosis, and/or chronic tubulointerstitial sclerosis; subjects having an ultrasound, MRI, CAT scan, or other non-invasive examination indicating renal fibrosis or smaller than normal kidneys. Further indications of subjects in, or at risk of CRF, are well known to workers having ordinary skill in the art. For example, all the following may be criteria to determine if a subject is in, or at risk for CRF: subjects having an unusual number of broad casts present in urinary sediment; subjects having a GFR which is chronically less than about 50%, and more particularly less than about 40%, 30% or 20%, of the expected GFR for the subject; human male subjects weighing at least about 50 kg and having a GFR which is chronically less than about 50 ml/min, and more particularly less than about 40 ml/min, 30 ml/min or 20 ml/min; human female subjects weighing at least about 40 kg and having a GFR which is chronically less than about 40 ml/min, and more particularly less than about 30 ml/min, 20 ml/min or 10 ml/min; subjects possessing a number of functional nephron units which is less than about 50%, and more particularly less than about 40%, 30% or 20%, of the number of functional nephron units possessed by a healthy but otherwise similar subject; subjects which have a single kidney; and subjects which are kidney transplant recipients.

A "therapeutic composition" as used herein is defined as comprising the proteins of the invention and other biologically compatible ingredients. The therapeutic composition may contain excipients such as water, minerals and carriers such as protein.

An antagonist of the invention is said to have "therapeutic efficacy," and an amount of the agent is said to be "therapeutically effective," if administration of that amount of the agent is sufficient to cause a clinically significant improvement in a standard marker of renal function when administered to a subject (e.g., an animal model or human patient) in, or at risk of, chronic renal failure. Such markers of renal function are well known in the medical literature and include, without being limited to, rates of increase in BUN levels, rates of increase in serum creatinine, static measurements of BUN, static measurements of serum creatinine, glomerular filtration rates (GFR), ratios of BUN/creatinine, serum concentrations of sodium (Na+), urine/plasma ratios for creatinine, urine/plasma ratios for urea, urine osmolality, daily urine output, and the like (see, for example, Brenner and Lazarus (1994), in Harrison's Principles of Internal Medicine, 13th edition, Isselbacher et al., eds., McGraw Hill Text, New York; Luke and Strom (1994), in Internal Medicine, 4th Edition, J.H. Stein, ed., Mosby-Year Book, Inc. St. Louis.)

Glomerular Filtration Rate (GFR). The "glomerular filtration rate" or "GFR" is proportional to the rate of clearance into urine of a plasma-borne substance which is not bound by serum proteins, is freely filtered across glomeruli, and is neither secreted nor reabsorbed by the renal tubules. Thus, as used herein, GFR preferably is defined by the following equation:

$$GFR = \frac{U_{conc} \times V}{P_{conc}}$$

where $U_{conc}$ is the urine concentration of the marker, $P_{conc}$ is the plasma concentration of the marker, and V is the urine flow rate in ml/min. Optionally, GFR is corrected for body surface area. Thus, the GFR values used herein may be regarded as being in units of ml/min/1.73m$^2$.

The preferred measure of GFR is the clearance of inulin but, because of the difficulty of measuring the concentrations of this substance, the clearance of creatinine is typically used in clinical settings. For example, for an average size, healthy human male (70 kg, 20-40 yrs), a typical GFR measured by creatinine clearance is expected to be approximately 125 ml/min with plasma concentrations of creatinine of 0.7-1.5 mg/dL. For a comparable, average size woman, a typical GFR measured by creatinine clearance is expected to be approximately 115 ml/min with creatinine levels of 0.5-1.3 mg/dL. During times of good health, human GFR values are relatively stable until about age 40, when GFR typically begins to decrease with age. For subjects surviving to age 85 or 90, GFR may be reduced to 50% of the comparable values at age 40.

Expected Glomerular Filtration Rate (GER_exp). An estimate of the "expected GFR" or "GFR_exp" may be provided based upon considerations of a subject's age, weight, sex, body surface area, and degree of musculature, and the plasma concentration of some marker compound (e.g., creatinine) as determined by a blood test. Thus, as an example, an expected GFR or $GFR_{exp}$ may be estimated as:

$$GFR_{exp} \approx \frac{(140 - age) \times weight\,(kg)}{72 \times P_{conc}\,(mg/dl)}$$

This estimate does not take into consideration such factors as surface area, degree of musculature, or percentage body fat. Nonetheless, using plasma creatinine levels as the marker, this formula has been employed for human males as an inexpensive means of estimating GFR. Because creatinine is produced by striated muscle, the expected GFR or $GFR_{exp}$ of human female subjects is estimated by the same equation multiplied by 0.85 to account for expected differences in muscle mass. (See Lemann, et al. (1990) Am. J. Kidney-Dis. 16(3):236-243.)

"wild type" is the naturally-occurring polynucleotide sequence of an exon of a protein, or a portion thereof, or protein sequence, or portion thereof, respectively, as it normally exists *in vivo.*

Practice of the present invention will employ, unless indicated otherwise, conventional techniques of cell biology, cell culture, molecular biology, microbiology, recombinant DNA, protein chemistry, and immunology, which are within the skill of the art. Such techniques are described in the literature. Unless stipulated otherwise, all references cited in the Detailed Description are incorporated herein by reference.

Description of the Preferred Embodiments

General

The present invention depends, in part, upon the discovery that efficacy of certain integrin antagonists to subjects in, or at risk of, acute renal failure, is not predictive of efficacy in chronic renal failure. We summarize these findings in the Examples. Moreover, we have found that certain integrin antagonists can reduce mortality and/or morbidity rates, and/or prevent, inhibit, delay or alleviate the progressive loss of renal function which characterizes chronic renal failure. Briefly, we developed an animal model of kidney disease having two phases- a first phase of immune mediated kidney inflammation (acute crescentic glomerulonephritis) lasting about 10 days, followed by a secondary tubulointerstitial nephritis phase accompanied by progressive glomerulosclerosis lasting until death from CRF around day 40. We found that antagonists to TGFbeta (which is a known mediator of fibrosis in CRF and is expressed after day 11 in this model) had no efficacy in the animal model during the acute phase, but was efficacious in the chronic phase. We found similar uncoupling between the acute and chronic phases using an anti VLA-1 antibody. Thus, one would not expect to find antagonists to alpha4 integrins with efficacy in both phases

**A. Integrin Antagonists**

For the purposes of the invention, an integrin antagonist can be an antagonist of any interaction between an integrin and its cognate ligand or receptor. One preferred embodiment of an integrin antagonist is an antagonist of interactions of alpha4 integrins with their ligands, such as the VCAM-1/VLA-4 interaction or the MadCAM/alpha4beta7 interaction (see Lobb and Hemler, *supra).* This is an agent, e.g., a polypeptide or other molecule, which can inhibit or block VCAM- 1 and/or VLA-4-mediated binding or which can otherwise modulate VCAM- 1 and/or VLA-4 function, e.g:, by inhibiting or blocking VLA-4-ligand mediated VLA-4 signal transduction or VCAM- 1 ligand mediated VCAM-1 signal transduction and which is effective in the treatment of chronic renal failure, preferably in the same manner as are anti-VLA-4 antibodies. Similarly, it is an agent e.g., a polypeptide or other molecule, which can inhibit or block MadCAM and/or alpha4beta7-mediated binding or which can otherwise modulate MadCAM and/or alpha4 beta7 function, e.g., by inhibiting or blocking alpha4 beta7-ligand mediated alpha4 beta 7 signal transduction or MadCAM-ligand mediated MadCAM signal transduction and which is effective in the treatment of chronic renal failure.

An antagonist of, for example, the VCAM- 1/ VLA-4 interaction is an agent which has one or more of the following properties: (1) it coats, or binds to, VLA-4 on the surface of a VLA-4 bearing cell (i.e., a lymphocyte) with sufficient specificity to inhibit a VLA-4 ligand/VLA-4 interaction, e.g., the VCAM-1/VLA-4 interaction; (2) it coats, or binds to, VLA-4 on the surface of a VLA-4 bearing cell (i.e., a lymphocyte) with sufficient specificity to modify, and preferably to inhibit, transduction of a VLA-4-mediated signal e.g., VLA-4NCAM- 1 mediated signaling; (3) it coats, or binds to, a VLA-4-ligand, (e.g., VCAM- 1) on endothelial cells with sufficient specificity to inhibit the VLA- 4/VCAM- 1 interaction; (4) it coats, or binds to, a VLA-4-ligand (e.g., VCAM- 1) with sufficient specificity to modify, and preferably to inhibit, transduction of VLA-4-ligand mediated VLA-4 signaling, e.g., VCAM-1-mediated VLA-4 signaling. In preferred embodiments, the antagonist has one or both of properties 1 and 2. In other preferred embodiments the antagonist has one or both of properties 3 and 4. Moreover, more than one antagonist can be administered to a patient, e.g., an agent which binds to VLA-4 can be combined with an agent which binds to VCAM-1.

As discussed herein, the antagonists used in methods of the invention are not limited to a particular type or structure of molecule so that, for purposes of the invention, any agent capable of binding to alpha4 integrins (e.g., VLA-4 or alpha4beta7) on the surface of cells or to an alpha4 ligand such as VCAM- 1 or MadCAM on the surface of alpha4 ligand-bearing cells) and which effectively blocks or coats alpha 4 integrin (e.g., VLA-4 or alpha4beta7) or alpha 4 ligand (e.g., VCAM- 1 or MadCAM, respectively), an "alpha4 integrin binding agent" and "alpha4 integrin ligand binding agent" respectively), is considered to be an equivalent of the antagonists used in the examples herein.

For example, antibodies or antibody homologs (discussed above) as well as soluble forms of the natural binding proteins for VLA-4 and VCAM-1 are useful. Soluble forms of the natural binding proteins for VLA-4 include soluble VCAM-I peptides, VCAM- I fusion proteins, bifunctional VCAM-1/Ig fusion proteins (e.g. "chimeric" molecules, discussed above), fibronectin, fibronectin having an alternatively spliced non-type III connecting segment, and fibronectin peptides containing the amino acid sequence EBLDV or a similar conservatively substituted amino acid sequence. Soluble forms of the natural binding proteins for VCAM- 1 include soluble VLA-4 peptides, VLA-4 fusion proteins, bifunctional VLA-4/Ig fusion proteins and the like. As used herein, a "soluble VLA-4 peptide" or a "soluble VCAM- 1 peptide" is a VLA-4 or VCAM-1 polypeptide incapable of anchoring itself in a membrane. Such soluble polypeptides include, for example, VLA-4 and VCAM polypeptides that lack a sufficient portion of their membrane spanning domain to anchor the polypeptide or are modified such that the membrane spanning domain is non-functional. These binding agents can act by competing with the cell-surface binding protein for VLA-4 or by otherwise altering VLA-4 function. For example, a soluble form of VCAM-1 (see, e.g., Osborn et al. 1989, Cell, 59: 1203-1211) or a fragment thereof may be administered to bind to VLA-4, and preferably compete for a VLA-4 binding site on VCAM- 1 bearing cells, thereby leading to effects similar to the administration of antagonists such as small molecules or anti-VLA-4 antibodies.

For the purposes of the invention an "antagonist of the collagen/VLA-1 interaction" refers to an agent, e.g., a polypeptide or other molecule, which can inhibit or block collagen and/or VLA-1 -mediated binding or which can otherwise modulate collagen and/or VLA=1 function, e.g., by inhibiting or blocking VLA- 1 -mediated VLA- 1 signal tralisduction or collagen-mediated collagen signal transduction and which is effective in the treatment of chronic renal failure, preferably in the same manner as are anti-VLA- 1 antibodies. An antagonist of the VLA-1 ligand (e.g. collagen)/ VLA- linteraction is an agent which has one or more of the following properties: (1) it coats, or binds to, VLA- 1 on the surface of a VLA-1 bearing cell with sufficient specificity to inhibit a collagen/VLA-1 interaction; (2) it coats, or binds to, VLA- 1 on the surface of a VLA- 1 bearing cell with sufficient specificity to modify, and preferably to inhibit, transduction of a VLA- 1-mediated signal e.g., VLA- 1/collagen-mediated signaling; (3) it coats, or binds to a VLA-1-ligand, (e.g., collagen) with sufficient specificity to inhibit the collagen/VLA- 1 interaction; (4) it coats, or binds to collagen with sufficient specificity to modify, and preferably to inhibit, transduction of collagen-mediated VLA- 1 signaling. In preferred embodiments the antagonist has one or both of properties 1 and 2. In other preferred embodiments, the antagonist has one or both of properties 3 and 4. Moreover, more than one antagonist can be administered to a patient, e.g., an agent which binds to VLA-1 can be combined with an agent which binds to collagen. As discussed herein, the VLA- 1 antagonists used in methods of the invention are not limited to a particular type or structure of molecule so that, for purposes of the invention, any agent capable of binding to VLA- 1 on the surface of VLA- 1 bearing cells (or to collagen) and which effectively blocks or coats VLA-1 or its ligand(s) is considered to be an equivalent of the VLA- 1 antagonists used in the examples herein. For example, antibodies or antibody homologs (discussed below) as well as soluble forms of the natural binding proteins for VLA-1 and their respective ligands are useful. Soluble forms of the natural binding proteins for VLA- I include soluble collagen peptides, collagen fusion proteins, bifunctional collagen/Ig fusion proteins, or a similar conservatively substituted amino acid sequence. Soluble forms of the natural binding proteins for collagen include soluble VLA- 1 peptides, VLA- 1 fusion proteins, bifunctional VLA-1/Ig fusion proteins and the like. As used herein, a "soluble VLA- 1 peptide" or a "soluble collagen peptide" is an VLA- 1 or collagen polypeptide incapable of anchoring itself in a membrane Such soluble polypeptides include, for example, VLA-1 polypeptides that lack a sufficient portion of their membrane spanning domain to anchor the polypeptide or are modified such that the membrane spanning domain is non-functional. These binding agents can act by competing with the cell-surface binding protein for VLA- 1 or by otherwise altering VLA- 1 function.

In another example, collagen or a fragment thereof which is capable of binding to VLA- 1 on the surface of VLA- 1 bearing cells can be linked or otherwise fused to a second moiety to form a VLA- 1 antagonist chimeric molecule. The second moiety can be a peptide, a fragment of a soluble peptide, preferably a human peptide, more preferably a plasma protein, or a member of the immunoglobulin superfamily. In particularly preferred embodiments, the second peptide is an IgG or a portion or fragment thereof, e.g., the human IgG1 heavy chain constant region and includes, at least the hinge, CH2 and CH3 domains.

1. Anti-Integrin Antibody Homologs

In other preferred embodiments, the antagonists used in the method of the invention to bind to, including block or coat, cell-surface alpha4 integrin (such as VLA-4) and/or cell surface ligand for alpha 4 integrin (such as VCAM- 1) is an anti-VLA-4 and/or anti-VCAM- 1 monoclonal antibody or antibody homolog, as defined previously. Preferred antibodies and homologs for treatment, in particular for human treatment, include human antibody homologs, humanized antibody homologs, chimeric antibody homologs, Fab, Fab', F(ab')2 and F(v) antibody fragments, and monomers or dimers of antibody heavy or light chains or mixtures thereof. Monoclonal antibodies against VLA-4 and/or VLA-1 are a preferred binding agent in the method of the invention.

2. Small Molecule Integrin Antagonists

The term small molecule" integrin antagonist refers to agents that mimic the action of peptides (i.e., organic molecules that may or may not contain peptide bonds) capable of disrupting the integrin/integrin ligand interaction by, for instance, blocking $VL_A$-4 by binding VLA-4 receptors on the surface of cells or blocking VCAM- 1 by binding VCAM- 1 receptors on the surface of cells. VLA-4 and VCAM- 1 (or VLA- 1 and coliagen) small molecules may themselves be peptides, semi-peptidic compounds or non-peptidic compounds, such as small organic molecules that are antagonists of the VCAM-1/VLA-4 and/or collagen/VLA-1 interaction. A "small molecule", as defined herein, is not intended to encompass an antibody or antibody homolog. The molecular weight of exemplary small molecules is generally less than about 2000 although we do not intend to be limited to any particular size range provided that the molecule meets the functional definition.

For instance, small molecules such as oligosaccharides that mimic the binding domain of a VLA-4 ligand and fit the receptor domain of VLA-4 may be employed. (See, J.J. Devlin et al., 1990, Science 249: 400-406 (1990), J.K. Scott and G.P. Smith, 1990, Science 249: 386-390, and U.S. Patent 4,833,092 (Geysen), all incorporated herein by reference. Conversely, small molecules that mimic the binding domain of a VCAM- 1 ligand and fit the receptor domain of VCAM- 1 maybe employed.

Examples of other small molecules useful in the invention can be found in Komoriya et al. ("The Minimal Essential Sequence for a Major Cell Type-Specific Adhesion Site (CS1) Within the Alternatively Spliced Type III Connecting Segment Domain of Fibronectin Is Leucine-Aspartic Acid-Valine", J. Biol. Chem., 266 (23), pp. 15075-79 (1991) ). They identified the minimum active amino acid sequence necessary to bind VLA-4 and synthesized a variety of overlapping peptides based on the amino acid sequence of the CS-1 region (the VLA-4 binding domain) of a particular species of fibronectin. They identified an 8-amino acid peptide, Glu-Ile-Leu-Asp-Val-Pro-Ser-Thr, as well as two smaller overlapping pentapeptides, Glu-Ile-Leu-Asp-Val and Leu-Asp-Val-Pro-Ser, that possessed inhibitory activity against fibronectin-dependent cell adhesion. Certain larger peptides containing the LDV sequence were subsequently shown to be active in vivo (T. A. Ferguson et al., "Two Integrin Binding Peptides Abrogate T-cell Mediated Immune Responses In Vivo", Proc. Natl. Acad. Sci. USA, 88, pp. 8072-76 (1991); and S. M. Wahl et al., "Synthetic Fibronectin Peptides Suppress Arthritis in Rats by Interrupting Leukocyte Adhesion and Recruitment", J. Clin. Invest., 94, pp. 655-62 (1994) ). A cyclic pentapeptide; Arg-Cys-Asp-TPro-Cys (wherein TPro denotes 4-thioproline), which can inhibit both VLA-4 and VLA-5 adhesion to fibronectin has also been described. (See, e.g., D.M. Nowlin et al. "A Novel Cyclic Pentapeptide Inhibits Alpha4Betal Integrin-mediated Cell Adhesion", J. Biol. Chem., 268(27), pp. 20352-59 (1993); and PCT publication PCT/US91/04862). This pentapeptide was based on the tripeptide sequence Arg-Gly-Asp from fibronectin which had been known as a common motif in the recognition site for several extracellular-matrix proteins. Examples of other VLA-4 inhibitors have been reported, for example, in Adams et al. "Cell Adhesion Inhibitors", PCT US97/13013, describing linear peptidyl compounds containing beta-amino acids which have cell adhesion inhibitory activity. Internatiorial patent applications WO 94/15958 and WO 92/00995 describe cyclic peptide and peptide mimetic compounds with cell adhesion inhibitory activity. International patent publications WO 93/08823 and WO 92/08464 describe guanidinyl-, urea- and thiourea-containing cell adhesion inhibitory compounds. United States Patent No. 5,260,277 describes guanidinyl cell adhesion modulation compounds. Other peptidyl antagonists of VLA-4 have been described in D. Y. Jackson et al., "Potent a4betal peptide antagonists as potential anti-inflammatory agents', J. Med. Chem., 40, 3359 (1997); H. Shroff et al., Small peptide Inhibitors of alpha4beta7 mediated MadCAM-1 adhesion to lymphocytes", Bio. Med, Chem. Lett., 1: 2495 (1996); U.S Patent 5,510,332, PCT Publications WO 98/53814, W097/03094, W097/02289, W096/40781, W096/22966,WO96/20216, W096/01644, W096106108, and W095/15973.

Such small molecule agents may be produced by synthesizing a plurality of peptides (e.g., 520 amino acids in length), semi-peptidic compounds or non-peptidic, organic compounds, and then screening those compounds for their ability to inhibit the VLA-4/VCAM interaction. See generally U.S. Patent No. 4,833,092, Scott and Smith, "Searching for Peptide Ligands with an Epitope Library", Science, 249, pp. 386-90 (1990), and Devlin et al., "Random Peptide Libraries: A Source of Specific Protein Binding Molecules", Science, 249, pp. 40407 (1990).

## Methods of Making Anti-Integrin Antibody Homologs

The technology for producing monoclonal antibodies, including for example, anti-integrin monoclonal antibodies is well known. See for example, Mendrick et al. 1995, Lab. Invest. 72:367-375 (mAbs to murine anti-α1β1 and anti-α2β1); Sonnenberg et al. 1987 J. Biol. Chem.262:10376-10383 (mAbs to murine anti-α6β1); Yao et al. 1996, J Cell Sci 1996 109:3139-50 (mAbs to murine anti-α7β1); Hemler et al. 1984, J Immunol 132:3011-8 (mAbs to human α1β1); Pischel et al. 1987 J Immunol 138:226-33 (mAbs to human α2β1); Wayner et al. 1988, J Cell Biol 107:1881-91 (mAbs to human α3β1); Hemler et al. 1987 J Biol Chem 262:11478-85 (mAbs to human α4β1); Wayner et al. 1988 J Cell Biol 107:1881-91 (mAbs to human α5β1); Sonnenberg et al. 1987, J. Biol. Chem. 262:10376-10383 (mAbs to human α6β1); A Wang et al. 1996 Am, J. Respir. Cell Mol. Biol. 15:664-672 (mAbs to human α9β1); Davies et al. 1989 J Cell Biol 109: 1817-26 (mAbs to human αV β1); Sanchez-Madrid et al. 1982, Proc Natl Acad Sci U S A 79:7489-93 (mAbs to human αL β2); Diamond et al. 1993, J Cell Biol 120:1031-43 (mAbs to human αMβ2); Stacker et al. 1991 J Immunol 146:648-55 (mAbs to human αXβ2); Van der Vieren et al 1995 Immunity 3:683-90 (mAbs to human αDβ2); Bennett et al. 1983 Proc Natl Acad Sci U S A 80:2417-21 (mAbs to human αIIbβ3); Hessle et al. 1984, Differentiation 26:49-54 (mAbs to human α6β4); Weinacker et al. 1994 J Biol Chem 269:6940-8 (mAbs to human αVβ5); Weinacker et al. 1994 J Biol Chem 269: 6940-8 (mAbs to human aVp6); Cerf-Bensussan et al 1992 Eur J Immunol 22:273-7 (mAbs to human αEβ7); Nishimura et al. 1994 J Biol Chem 269:28708-15 (mAbs to human αVβ8); Bossy et al. 1991 EMBO J 10:2375-85 (polyclonal antisera to human α8β1); Camper et al. 1998 J. Biol. Chem. 273:20383-20389 (polyclonal antisera to human α10β1).

The preferred integrin antagonists contemplated herein can be expressed from intact or truncated genomic or cDNA or from synthetic DNAs in prokaryotic or eukaryotic host cells. The dimeric proteins can be isolated from the culture media and/or refolded and dimerized in vitro to form biologically active compositions. Heterodimers can be formed in vitro by combining separate, distinct polypeptide chains. Alternatively, heterodimers can be formed in a single cell by co-expressing nucleic acids encoding separate, distinct polypeptide chains. See, for example, W093/09229, or U.S. Pat. No. 5,411,94 1, for several exemplary recombinant heterodimer protein production protocols. Currently preferred host cells include, without limitation, prokaryotes including E. coli, or eukaryotes including yeast, Saccharomyces, insect cells, or mammalian cells, such as CHO, COS or BSC cells. One of ordinary skill in the art will appreciate that other host cells can be used to advantage. Detailed descriptions of the proteins useful in the practice of this invention, including how to make, use and test them for chondrogenic activity, are disclosed in numerous publications, including U.S. Pat. Nos. 5,266,683 and 5,011,69 1, the disclosures of which are herein incorporated by reference.

The technology for producing monoclonal antibody homologs is well known. Briefly, an immortal cell line (typically myeloma cells) is fused to lymphocytes (typically splenocytes) from a mammal immunized with whole cells expressing a given antigen, e.g., VLA-4, and the culture supernatants of the resulting hybridoma cells are screened for antibodies against the antigen. See, generally, Kohler et at., 1975, Nature, 265: 295-297. Immunization may be accomplished using standard procedures.. The unit dose and immunization regimen depend on the species of mammal immunized, its immune status, the body weight of the mammal, etc. Typically, the immunized mammals are bled and the serum from each blood sample is assayed for particular antibodies using appropriate screening assays. For example, anti-VLA-4 antibodies may be identified by immunoprecipitation of 125I-labeled cell lysates from VLA-4-expressing cells. (See, Sanchez-Madrid et al. 1986, Eur. ,J. Immunol., 16: 1343-1349 and Hemler et al. 1987, J. .Biol. Chem., 262, 11478-11485). Anti-VLA-4 antibodies may also be identified by flow cytometry, e.g., by measuring fluorescent staining of'Ramos cells incubated with an antibody believed to recognize VLA-4 (see, Elices et al., 1990, Cell, 60: 577-584). The lymphocytes used in the production of hybridoma cells typically are isolated from immunized mammals whose sera have already tested positive for the presence of anti-VLA-4 antibodies using such screening assays.

Typically, the immortal cell line (e.g., a myeloma cell line) is derived from the same mammalian species as the lymphocytes. Preferred immortal cell lines are mouse myeloma cell lines that are sensitive to culture medium containing hypoxanthine, aminopterin and thymidine ("HAT medium"). Typically, HAT-sensitive mouse myeloma cells are fused to mouse splenocytes using 1500 molecular weight polyethylene glycol ("PEG 1500"). Hybridoma cells resulting from the fusion are then selected using HAT medium, which kills unfused and unproductively fused myeloma cells (unfused splenocytes die after several days because they are not transformed). Hybridomas producing a desired antibody are detected by screening the hybridoma culture supernatants. For example, hybridomas prepared to produce anti-VLA-4 antibodies may be screened by testing the hybridoma culture supernatant for secreted antibodies having the ability to bind to a recombinant alpha4-subunit-expressing cell line (see, Elices et al., supra).

To produce anti-VLA-4 or VLA- 1 antibody homologs that are intact immunoglobulins, hybridoma cells that tested positive in such screening assays were cultured in a nutrient medium under conditions and for a time sufficient to allow the hybridoma cells to secrete the monoclonal antibodies into the culture medium. Tissue culture techniques and culture media suitable for hybridoma cells are well known. The conditioned hybridoma culture supernatant may be collected and the anti-VLA-4 or VLA-1 antibodies optionally further purified by well-known methods.

Alternatively, the desired antibody may be produced by injecting the hybridoma cells into the peritoneal cavity of an unimmunized mouse. The hybridoma cells proliferate in the peritoneal cavity, secreting the antibody which accumulates as ascites fluid. The antibody may be harvested by withdrawing the ascites fluid from the peritoneal cavity with a syringe.

Several mouse anti-VLA-4 monoclonal antibodies have been previously described. See, e.g., Sanchez-Madrid et al., 1986, supra; Hemler et al., 1987, supra; Pulido et al., 1991, J. Biol. Chem., 266 (16), 10241-10245). These anti-VLA-4 monoclonal antibodies such as HP 1/2 and other anti-VLA-4 antibodies (e.g., HP2/1, HP2/4, L25, P4C2, P4G9) capable of recognizing the beta chain of VLA-4 will be useful in the methods of treatment according to the present invention. Anti VLA-4 antibodies that will recognize the VLA-4 alpha4 chain epitopes involved in binding to VCAM- 1 and fibronectin ligands (i.e., antibodies which can bind to VLA-4 at a site involved in ligand recognition and block VCAM-1 and fibronectin binding) are preferred. Such antibodies have been defined as B epitope-specific antibodies (B1 or B2) (Pulido et al., 1991, supra) and are also anti-VLA-4 antibodies according to the present invention. Commercially available antibody homologs of the invention that are anti-VLA 1 antibodies have been described in U.S. 5,855,888.

Fully human monoclonal antibody homologs against VLA-4 and/or VLA-1 are another preferred binding agent which may block or coat VLA-4 and/or VLA-1 ligands in the method of the invention. In their intact form these may be prepared using in vitro-prinied human splenocytes, as described by Boemer et al., 1991, J. Immunol., 147, 86-95. Alternatively, they may be prepared by repertoire cloning as described by Persson et al., 1991, Proc. Nat. Acad Sci.USA, 88: 2432-2436 or by Huang and Stollar, 1991, J. Immunol. Methods 141, 227-236.

U.S. Patent 5,798,230 (Aug. 25, 1998, "Process for the preparation of human monoclonal antibodies and their use") describes preparation of human monoclonal antibodies from human B cells. According to this process, human antibody-producing B cells are immortalized by infection with an Epstein-Barr virus, or a derivative thereof, that expresses Epstein-Barr virus nuclear antigen 2 (EBNA2). EBNA2 function, which is required for immortalization, is subsequently shut off, which results in an increase in antibody production.

In yet another method for producing fully human antibodies, United States Patent 5,789,650 (Aug. 4, 1998, "Transgenic non-human animals for producing Heterologous antibodies") describes transgenic non-human animals capable of producing heterologous antibodies and transgenic non-human animals having inactivated endogenous immunoglobulin genes. Endogenous immunoglobulin genes are suppressed by antisense polynucleotides and/or by antiserum directed against endogenous immunoglobulins. Heterologous antibodies are encoded by immunoglobulin genes not normally found in the genome of that species of non-human animal. One or more transgenes containing sequences of unrearranged heterologous human immunoglobulin heavy chains are introduced into a non-human animal thereby forming a transgenic animal capable of functionally rearranging transgenic immunoglobulin sequences and producing a repertoire of antibodies of various isotypes encoded by human immunoglobulin genes. Such heterologous human antibodies are produced in B-cells which are thereafter immortalized, e.g., by fusing with an immortalizing cell line such as a myeloma or by manipulating such B-cells by other techniques to perpetuate a cell line capable of producing a monoclonal heterologous, fully human antibody homolog.

Large nonimmunized human phage display libraries may also be used to isolate high affinity antibodies that can be developed as human therapeutics using standard phage technology

Yet another preferred binding agent which may block or coat integrin ligands in the method of the invention is a humanized recombinant antibody homolog having anti-integrin specificity. Following the early methods for the preparation of true "chimeric antibodies" (where the entire constant and entire variable regions are derived from different sources), a new approach was described in EP 0239400 (Winter et al.) whereby antibodies are altered by substitution (within a given variable region) of their complementarity determining regions (CDRs) for one species with those from another. This process may be used, for example, to substitute the CDRs from human heavy and light chain Ig variable region domains with alternative CDRs from murine variable region domains. These altered Ig variable regions may subsequently be combined with human Ig constant regions to created antibodies which are totally human in composition except for the substituted murine CDRs. Such CDR-substituted antibodies would be predicted to be less likely to elicit an immune response in humans compared to true chimeric antibodies because the CDR-substituted antibodies contain considerably less non-human components. The process for humanizing monoclonal antibodies via CDR "grafting" has been termed "reshaping". (Riechmann et al., 1988, Nature 332, 323-327; Verhoeyen et al., 1988, Science 239, 1534-1536).

Typically, complementarity determining regions (CDRs) of a murine antibody are transplanted onto the corresponding regions in human antibody, since it is the CDRs (three in antibody heavy chains, three in light chains) that are the regions of the mouse antibody which bind to a specific antigen. Transplantation of CDRs is achieved by genetic engineering whereby CDR DNA sequences are determined by cloning of murine heavy and light chain variable (V) region gene segments, and are then transferred to corresponding human V regions by site directed mutagenesis. In the final stage of the process, human constant region gene segments of the desired isotype (usually gamma I for CH and kappa for CL) are added and the humanized heavy and light chain genes are co-expressed in mammalian cells to produce soluble humanized antibody.

The transfer of these CDRs to a human antibody confers on this antibody the antigen binding properties of the original murine antibody. The six CDRs in the murine antibody are mounted structurally on a V region "framework" region.

The reason that CDR-grafting is successful is that framework regions between mouse and human antibodies may have very similar 3-D structures with similar points of attachment for CDRs, such that CDRs can be interchanged. Such humanized antibody homologs may be prepared, as exemplified in Jones et al., 1986, Nature 321, 522-525; Riechmann, 1988, Nature 332, 323-327; Queen et al., 1989, Proc. Nat. Acad. Sci. USA 86, 10029; and Orlandi et a]., 1989, Proc. Nat. Acad. Sci. USA 86, 3833.

Nonetheless, certain amino acids within framework regions are thought to interact with CDRs and to influence overall antigen binding affinity. The direct transfer of CDRs from a murine antibody to produce a recombinant humanized antibody without any modifications of the human V region frameworks often results in a partial or complete loss of binding affinity. In a number of cases, it appears to be critical to alter residues in the framework regions of the acceptor antibody in order to obtain binding activity.

Queen et al., 1989 (supra) and WO 90/07861 (Protein Design Labs) have described the preparation of a humanized antibody that contains modified residues in the framework regions of the acceptor antibody by combining the CDRs of a murine MAb (anti-Tac) with human immunoglobulin framework and constant regions. They have demonstrated one solution to the problem of the loss of binding affinity that often results from direct CDR transfer without any modifications of the human V region framework residues; their solution involves two key steps. First, the human V framework regions are chosen by computer analysts for optimal protein sequence homology to the V region framework of the original murine antibody, in this case, the anti-Tac MAb. In the second step, the tertiary structure of the murine V region is modeled by computer in order to visualize framework amino acid residues which are likely to interact with the murine CDRs and these murine amino acid residues are then superimposed on the homologous human framework. See also Protein Design Labs U.S. Patent 5,693,762.

One may use a different approach (Tempest et al., 1991, Biotechnology 9, 266-27 1) and utilize, as standard, the V region frameworks derived from NEWM and REI heavy and light chains respectively for CDR-grafting without radical introduction of mouse residues. An advantage of using the Tempest et al., approach to construct NEWM and REI based humanized antibodies is that the 3-dimensional structures of NEWM and REI variable regions are known from x-ray crystallography and thus specific interactions between CDRs and V region framework residues can be modeled.

Regardless of the approach taken, the examples of the initial humanized antibody homologs prepared to date have shown that it is not a straightforward process. However, even acknowledging that such framework changes may be necessary, it is not possible to predict, on the basis of the available prior art, which, if any, framework residues will need to be altered to obtain functional humanized recombinant antibodies of the desired specificity. Results thus far indicate that changes necessary to preserve specificity and/or affinity are for the most part unique to a given antibody and cannot be predicted based on the humanization of a different antibody.

Preferred VLA-4 antagonists useful in the present invention include chimeric and humanized recombinant antibody homologs (i.e., intact immunoglobulins and portions thereof) with B epitope specificity that have been prepared and are described in co-pending U.S. Patent Application Serial No. 08/004,798, filed January 12, 1993, PCT US94/00266, filed January 7, 1994. The starting material for the preparation of chimeric (mouse Variable - human Constant) and humanized anti-integrin antibody homologs may be a murine monoclonal anti-integrin antibody as previously described, a monoclonal anti-integrin antibody commercially available (e.g., HP2/1, Amae International, Inc., Westbrook, Maine), or a monoclonal anti-integrin antibody prepared in accordance with the teaching herein. For example, the variable regions of the heavy and light chains of the anti-VLA-4 antibody HP ½ have been cloned, sequenced and expressed in combination with constant regions of human immunoglobulin heavy and light chains. Such HP ½ antibody is similar in specificity and potency to the murine HP 1/2 antibody, and may be useful in methods of treatment according to the present invention.

Other preferred humanized anti-VLA4 antibody homologs are described by Athena Neurosciences, Inc. in PCT/US95/01219 (27 July 1995) and U.S. Patent 5,840,299 (incorporated herein by reference) These humanized anti-VLA-4 antibodies comprise a humanized light chain and a humanized heavy chain. The humanized light chain comprises three complementarity determining regions (CDR1, CDR2 and CDR3) having amino acid sequences from the corresponding complementarity determining regions of a mouse 21-6 immunoglobulin light chain and a variable region framework from a human kappa light chain variable region framework sequence except in at least position the amino acid position is occupied by the same amino acid present in the equivalent position of the mouse 21.6 immunoglobulin light chain variable region framework. The humanized heavy chain comprises three complementarity determining regions (CDR 1, CDR2 and CDR3) having amino acid sequences from the corresponding complementarity determining regions of a mouse 21-6 immunoglobulin heavy chain, and a variable region framework from a human heavy chain variable region framework sequence except in at least one position the amino acid position is occupied by the same amino acid present in the equivalent position of the mouse 21-6 immunoglobulin heavy chain variable region framework. See also Leger, O.J. et al., Hum. Antibodies. 8.3-16 (1997) describing a humanized antibody (AN 100226) against human alpha4 integrin.

The methods of the present invention may utilize antagonists encoded by any nucleic acid sequences that hybridize under stringent conditions to nucleic acid sequences encoding antibodies directed against alpha4 subunit containing integrins. For example, an antagonist of the present invention may be a protein encoded by a nucleic acid sequence that hybridizes under high stringency conditions to one or more of those nucleic acid sequences found in Table 6 of U.S. Patent 5,840,299 or the complement of such one or more sequences. Antagonists may also be a protein encoded by nucleic acid sequences that hybridize under high stringency conditions to a nucleic acid encoding SEQ ID NO: 2 or SEQ ID NO: 4 found in U.S. Patent 5,932,214. Further, antagonists may also be a protein encoded by nucleic acid sequences that hybridize under high stringency conditions to a nucleic acid encoding a variable domain of the antibody produced by cell line ATCC CRL 11175.

Alternatively, an antagonist of the present invention may be a protein encoded by a nucleic acid sequence that hybridizes under low stringency conditions to one or more of those nucleic acid sequence found in Table 6 of U.S. Patent 5,840,299 or the complement of such one or more sequences. Antagonists may also be a protein encoded by a nucleic acid sequence that hybridizes under low stringency conditions to a nucleic acid encoding SEQ ID NO: 2 or SEQ ID NO: 4 found in U.S. Patent 5,932,214. (Further, antagonists may also be a protein encoded by nucleic acid sequences that hybridize under low stringency conditions to a nucleic acid encoding a variable domain of the antibody produced by cell line ATCC CRL 11175.

**Therapeutic Applications**

The amount of active ingredient that may be combined with the carrier materials to produce a single dosage form will vary depending upon the host treated, and the particular mode of administration. It should be understood, however, that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of active ingredient may also depend upon the therapeutic or prophylactic agent, if any, with which the ingredient is co-administered.

The dosage and dose rate of the compounds of this invention effective to prevent, suppress or inhibit cell adhesion will depend on a variety of factors, such as the nature of the inhibitor, the size of the patient, the goal of the treatment, the nature of the pathology to be treated, the specific pharmaceutical composition used, and the judgment of the treating physician. Dosage levels of between about 0.001 and about 100 mg/kg body weight per day, preferably between about 0.1 and about 50 mg/kg body weight of the active ingredient compound are useful. Most preferably, the alpha4 integrin binding agent, if an antibody or antibody derivative, will be administered at a dose ranging between about 0.1 mg/kg body weight and about 20 mg/kg body weight, preferably ranging between about 1 mg/kg body weight and about 3 mg/kg body weight and at intervals of every 1-14 days. For non-antibody or small molecule binding agents, the dose range should preferably be between molar equivalent amounts to these amounts of antibody. Preferably, an antibody composition is administered in an amount effective to provide a plasma level of antibody of at least 1 mg/ml. Optimization of dosages can be determined by administration of the binding agents, followed by assessment of the coating of integrin-positive cells by the agent over time after administered at a given dose in vivo.

The presence of the administered agent may be detected in vitro (or ex vivo) by the inability or decreased ability of the individual's cells to bind the same agent which has been itself labeled (e.g., by a fluorochrome). The preferred dosage should produce detectable coating of the vast majority of integrin-positive cells. Preferably, coating is sustained in the case of an antibody homolog for a 1- 14 day period.

**Subjects for Treatment**

As a general matter, the methods of the present invention may be utilized for any mammalian subject in, or at risk of, chronic renal failure, or at risk of the need for renal replacement therapy (i.e., chronic dialysis or renal transplant). Mammalian subjects which may be treated according to the methods of the invention include, but are not limited to, human subjects or patients. In addition, however, the invention may be employed in the treatment of domesticated mammals which are maintained as human companions (e.g., dogs, cats, horses), which have significant commercial value (e.g., dairy cows, beef cattle, sporting animals), which have significant scientific value (e.g., captive or free specimens of endangered species), or which otherwise have value. In addition, as a general matter, the subjects for treatment with the methods of the present invention need not present indications for treatment with the renal therapeutic agents of the invention other than those indications associated with risk of chronic renal failure. That is, the subjects for treatment are expected to be otherwise free of indications for treatment with the agents of the invention. In some number of cases, however, the subjects may present with other symptoms (e.g., osteodystrophy) for which treatment with the agents of the present invention would be indicated. In such cases, the treatment should be adjusted accordingly so to avoid excessive dosing. One of ordinary skill in the medical or veterinary arts is trained to recognize subjects which may be at a substantial risk of chronic renal failure, or at substantial risk of the need for renal replacement therapy. In particular, clinical and non-clinical trials, as well as accumulated experience, relating to the presently disclosed and other methods of treatment, are expected to inform the skilled practitioner in deciding whether a given subject is in, or at risk of, chronic renal failure, or at risk of needing renal replacement therapy, and whether any particular treatment is best suited to the subject's needs, including treatment according to the present invention.

As a general matter, a mammalian subject may be regarded as being in, or at risk of, chronic renal failure, or at risk of needing renal replacement therapy, if that subject has already been diagnosed as afflicted with, or would be regarded as being afflicted with, a condition which typically leads to progressive loss of renal function associated with progressive loss of functioning nephron units. Such conditions include, but are not limited to, end-stage renal disease, chronic diabetic nephropathy, diabetic glomerulopathy, diabetic renal hypertrophy, hypertensive nephrosclerosis, hypertensive glomerulosclerosis, chronic glomerulonephritis, hereditary nephritis, renal dysplasia and chronic rejection following renal allograft transplantation and the like. These, and other diseases and conditions known in the art, typically lead to a progressive loss of functioning nephrons and to the onset of chronic renal failure.

Frequently, one of skill in the medical or veterinary arts may base a prognosis, diagnosis or treatment decision upon an examination of a renal biopsy sample. Such biopsies provide a wealth of information useful in diagnosing disorders of the kidney but, due to the invasiveness of the procedure, and the additional trauma to a presumably unhealthy kidney, may not be appropriate for all subjects. Nonetheless, subjects in, or at risk of, chronic renal failure, or at risk of needing renal replacement therapy, may be recognized by histological indications from renal biopsies including, but not limited to, glomerular hypertrophy, tubular hypertrophy, glomerulosclerosis, tubulointerstitial sclerosis, and the like.

Less invasive techniques for assessing kidney morphology include MRI, CAT and ultrasound scans. Scanning techniques are also available which employ contrasting or imaging agents (e.g., radioactive dyes) but, it should be noted, some of these are particularly toxic to renal tissues and structures and, therefore, their use may be ill-advised in subjects in, or at risk of, chronic renal failure. Such non-invasive scanning techniques may be employed to detect conditions such as renal fibrosis or sclerosis, focal renal necrosis, renal cysts, and renal gross hypertrophy which will place a subject in, or at risk of, chronic renal failure, or at risk of needing renal replacement therapy.

Frequently, prognosis, diagnosis and/or treatment decisions are based upon clinical indications of renal function. One such indication is the presence in urinary sediment of an unusual number of "broad" or "renal failure" casts, which is indicative of tubular hypertrophy and suggests the compensatory renal hypertrophy which typifies chronic renal failure. A better indication of renal function is the glomerular flow rate (GFR), which can be measured directly by quantifying the rate of clearance of particular markers, or which may be inferred from indirect measurements.

The methods of treatment of the present invention need not be restricted to subjects presenting with any particular measures of GFR, or any other particular marker of renal function. Indeed, it is not necessary that the GFR of a subject, or any other particular marker of renal function, be determined before practicing the treatments of the present invention. Nonetheless, the measurement of GFR is considered to be a preferred means of assessing renal function.

As is well known in the art, GFR reflects the rate of clearance of a reference or marker compound from the plasma to the urine. The marker compound to be considered is typically one which is freely filtered by the glomeruli, but which is not actively secreted or reabsorbed by the renal tubules, and which is not significantly bound by circulating proteins. The rate of clearance is typically defined by the formula, presented above, which relates the volume of urine produced in a twenty-four period, and the relative concentrations of the marker in the urine and plasma. To be more accurate, the GFR should also be corrected for body surface area. The "gold standard" reference compound is inulin because of its filtration properties and lack of serum-binding. The concentration of this compound is, however, difficult to quantify in blood or urine. The clearance rates of other compounds, including creatinine, are therefore often used instead of inulin. In addition, various formulas are often employed which seek to simplify the estimation of actual GFR by omitting considerations of actual urine concentrations of the marker, actual daily volumes of urine produced, or actual body surface area. These values may be replaced by estimates based on other factors, by baseline values established for the same subject, or by standard values for similar subjects. These estimates should be used with caution, however, as they may entail inappropriate assumptions based upon the renal function of normal or healthy subjects. In addition, clearance of p-aminohippurate (PAH) is used to estimate renal clearance rates.

Various methods and formulas have been developed in the art which describe an expected value of GFR for a healthy subject with certain characteristics. In particular, formulas are available which provide an expected value of the GFR based upon plasma creatinine levels, age, weight and sex. Other formulas may, of course, be employed and tables of standard values may be produced for subjects of a given age, weight, sex, and/or plasma creatinine concentration. Newer methods of measuring or estimating GFR (e.g., using NMR or MRI technologies) are also now available in the art and may be used in accordance with the present invention (see, e.g., U.S. Pat. Nos. 5,100,646 and 5,335,660).

As a general matter, irrespective of the manner in which GFR is measured or estimated, a subject may be considered to be in, or at risk of, chronic renal failure, or at risk of needing renal replacement therapy, when the subject has a GFR which is chronically less than about 50% of the expected GFR for that subject. The risk is considered greater as the GFR falls lower. Thus, a subject is increasingly considered at risk if the subject has a GFR which is chronically less than about 40%, 30% or 20% of the expected GFR. A human male subject weighing at least about 50 kg may be considered to be in, or at risk of, chronic renal failure, or at risk of needing renal replacement therapy, when the subject has a GFR which is chronically less than about 50 ml/min. The risk is considered greater as the GFR falls lower. Thus, a subject is increasingly considered at risk if the subject has a GFR which is chronically less than about 40, 30 or 20 ml/min. A human female subject weighing at least about 40 kg may be considered to be in, or at risk of, chronic renal failure, or at risk of needing renal replacement therapy, when the subject has a GFR which is chronically less than about 40 ml/min. The risk is considered greater as the GFR falls lower. Thus, a subject is increasingly considered at risk if the subject has a GFR which is chronically less than about 30, 20 or 10 ml/min. As a general matter, a subject may be regarded to be in, or at risk of, chronic renal failure, or at risk of needing renal replacement therapy, if that subject possesses a number of functional nephron units which is less than about 50% of the number of functional nephron units of a healthy, but otherwise similar, subject. As above, the risk is considered greater as the number of functional nephrons decreases further. Thus, a subject is increasingly considered at risk if the subject has a number of functional nephrons which is less than about 40, 30 or 20% of the number for a similar but healthy subject.

Finally, it should be noted that subjects possessing a single kidney, irrespective of the manner of loss of the other kidney (e.g., physical trauma, surgical removal, birth defect), may be considered to be prima facie at risk of chronic renal failure, or the need for renal replacement therapy. This is particularly true for those subjects in which one kidney has been lost due to a disease or condition which may afflict the remaining kidney. Similarly, subjects which are already recipients of a renal transplant, or which are already receiving chronic dialysis (e.g., chronic hemodialysis or continuous ambulatory peritoneal dialysis) may be considered to be at risk of chronic renal failure, or the need for further renal replacement therapy.

## Formulations and Methods of Treatment

The integrin antagonist agents of the present invention may be administered by any route which is compatible with the particular renal therapeutic agent employed. Thus, as appropriate, administration may be oral or parenteral, including intravenous, intraperitoneal, and renal intracapsular routes of administration. In addition, administration may be by periodic injections of a bolus of the agent(s) described herein, or may be made more continuous by intravenous or intraperitoneal administration from a reservoir which is external (e.g., an i.v. bag) or internal (e.g., a bioerodable implant or implanted pump). In a method according to the invention, integrin antagonists are preferably administered parenterally. The term "parenteral" as used herein includes aerosol, subcutaneous, intravenous, intramuscular, intra-articular, intrasynovial, intrasternal, intrathecal, intrahepatic, intralesional and intracranial injection or infusion techniques.

The agents of the invention may be provided to an individual by any suitable means, preferably directly (e.g., locally, as by injection or topical administration to a tissue locus) or systemically (e.g., parenterally or orally). Where the agent is to be provided parenterally, such as by intravenous, subcutaneous, or intramuscular, administration, the agent preferably comprises part of an aqueous solution. The solution is physiologically acceptable so that in addition to delivery of the desired agent to the subject, the solution does not otherwise adversely affect the subject's electrolyte and/or volume balance. The aqueous medium for the agent thus may comprise normal physiologic saline (e.g., 0.9% NaCl, 0.15M, pH 7-7.4).

The integrin antagonists are preferably administered as a sterile pharmaceutical composition containing a pharmaceutically acceptable carrier, which may be any of the numerous well known carriers, such as water, saline, phosphate buffered saline, dextrose, glycerol, ethanol, and the like, or combinations thereof. The compounds of the present invention may be used in the form of pharmaceutically acceptable salts derived from inorganic or organic acids and bases. Included among such acid salts are the following: acetate, adipate, alginate, aspartate, benzoate, benzenesulfonate, bisulfate, butyrate, citrate, camphorate, camphorsulfonate, cyclopentanepropionate, digluconate, dodecylsufate ethanesulfonate, fumarate, glucoheptanoate, glycerophosphate; hemisulfate, heptanoate, hexanoate, hydrochloride, hydrobromide, hydroiodide, 2-hydroxyethanesulfonate, lactate, maleate, methanesulfonate, 2naphthalenesulfonate, nicotinate, oxalate, pamoate, pectinate, persulfate, 3-phenyl-propionate, picrate, pivalate, propionate, succinate, tartrzlte, thiocyanate, tosylate and utidecanoate. Base salts include ammonium salts, alkali metal salts, such as sodium and potassium salts, alkaline earth metal salts, such as calcium and magnesium salts, salts with organic bases, such as dicyclohexylamine salts, N-inethyl-D-glucamine, tris(hydroxymethyl)methylamine and salts with amino acids such as arginine, lysine, and so forth. Also, the basic nitrogen-containing groups can be quatemized with such agents as lower alkyl halides, such as methyl, ethyl, propyl, and butyl chloride, bromides and iodides; dialkyl sulfates, such as dimethyl, diethyl, dibutyl and diamyl sulfates, long chain halides such as decyl, latiryl, myristyl and stearyl chlorides, bromides and iodides, aralkyl halides, such as benzyl and phenethyl bromides and others. Water or oil-soluble or dispersible products are thereby obtained.

The pharmaceutical compositions of this invention comprise any of the compounds of the present invention, or,pharmaceutically acceptable derivatives thereof, together with any pharmaceutically acceptable carrier. The term "carrier" as used herein includes acceptable adjuvants and vehicles. Pharmaceutically acceptable carriers that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as prolamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

According to this invention, the pharmaceutical compositions may be in the form of a sterile injectable preparation, for example a sterile injectable aqueous or oleaginous suspension. This suspension may be formulated according to techniques known in the art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or di-glycerides. Fatty acids, such as oleic acid and its glyceride derivatives are useful in the preparation of injectables, as do natural pharmaceutically-acceptable oils, such as olive oil or castor oil, especially in their polyoxyethylated versions. These oil solutions or suspensions may also contain a long-chain alcohol diluent or dispersant.

The pharmaceutical compositions of this invention, in particular small molecule integrin antagonists may be given parenterally or orally. If given orally, they can be administered in any orally acceptable dosage form including, but not limited to, capsules, tablets, aqueous suspensions or solutions. In the case of tablets for oral use, carriers which are commonly used include lactose.and corn starch. Lubricating agents, such as magnesium stearate, are also typically added. For oral administration in a capsule form, useful diluents include lactose and dried corn starch. When aqueous suspensions are required for oral use, the active ingredient is combined with emulsifying and suspending tgents. If desired, certain sweetening, flavoring or coloring agents may also be added. Topically-transdermal patches may also be used.

The pharmaceutical compositions of this invention may also be administered by nasal aerosol or inhalation through the use of a nebulizer, a dry powder inhaler or a metered dose inhaler. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents. According to another embodiment compositions containing a compound of this invention may also comprise an additional agent selected from the group consisting of corticosteroids, anti-inflammatories, immunosuppressants, anti-metabolites, and immunomodulators. Compounds within each of these classes may be selected from any of those listed under the appropriate group headings in "Comprehensive Medicinal Chemistry", Pergamon Press, Oxford, England; pp. 970-986 (1990), the disclosure of which is herein incorporated by reference. Specific compounds are theophylline, Sulfasalazine and aminosalicylates (anti-inflammatories); cyclosporin, FK-506, and rapamycin (immunosuppressants); cyclophosphamide and methotrexate (antimetabolites); steroids (inhaled , oral or topical) and interferons (immunomodulators),

Useful solutions for parenteral administration may be prepared by any of the methods well known in the pharmaceutical art, described, for example, in Remington's Pharmaceutical Sciences (Gennaro, A., ed.), Mack Pub., 1990. As will be appreciated by one of ordinary skill in the art, the formulated compositions contain therapeutically effective amounts of the renal therapeutic agent. That is, they contain amounts which provide appropriate concentrations of the agent to the renal tissues for a time sufficient to prevent, inhibit, delay or alleviate permanent or progressive loss of renal function, or otherwise provide therapeutic efficacy. As will be appreciated by those skilled in the art, the concentration of the compounds described in a therapeutic composition of the present invention will vary depending upon a number of factors, including the biological efficacy of the selected agent, the chemical characteristics (e.g., hydrophobicity) of the compounds employed, the formulation of the compound excipients, the administration route, and the treatment envisioned, including whether the active ingredient will be administered directly into a kidney or renal capsule, or whether it will be administered systemically. The preferred dosage to be administered also is likely to depend on such variables such as the condition of the renal tissues, extent of renal function loss, and the overall health status of the particular subject. Dosages may be administered continuously, or daily, but it, is currently preferred that dosages be administered once, twice or three times per week for as long as satisfactory response persists (as measured, for example, by stabilization and/or improvement of renal function by appropriate medical markers and/or quality of life indices) Less frequent dosages, for example monthly dosages, may also be employed. For subjects which would otherwise require continuous, bi-weekly or triweekly hemodialysis sessions, continuous, bi-weekly or triweekly intravenous or intraperitoneal infusions are not considered unduly inconvenient. In addition, in order to facilitate frequent infusions, implantation of a semi-permanent stent (e.g., intravenous, intraperitoneal or intracapsular) may be advisable.

The renal therapeutic agents of the invention may, of course, be administered alone or in combination with other molecules known to be beneficial in the treatment of the conditions described herein. When used in combination with other agents, it may be advisable to alter the dosages of the renal therapeutic agents of the present invention accordingly.

Animal Models

The agents of the present invention also may be tested in animal models of chronic renal failure. Mammalian models of chronic renal failure in, for example, mice, rats, guinea pigs, cats, dogs, sheep, goats, pigs, cows, horses, and non-human primates, may be created by causing an appropriate direct or indirect injury or insult to the renal tissues of the animal. Animal models of chronic renal failure may, for example, be created by performing a partial (e.g., 5/6) nephrectomy which reduces the number of functioning nephron units to a level which initiates compensatory renal hypertrophy, further nephron loss, and the progressive decline in renal function which characterizes chronic renal failure. Finally, the agents of the present invention may be evaluated for their therapeutic efficacy in causing a clinically significant improvement in a standard marker of renal function when administered to a mammalian subject (e.g., a human patient) in, or at risk of, chronic renal failure. Such markers of renal function are well known in the medical literature and include, without being limited to, rates of increase in BUN levels, rates of increase in serum creatinine, static measurements of BUN, static measurements of serum creatinine, glomerular filtration rates (GFR), ratios of BUN/creatinine, serum concentrations of sodium (Na+), urine/plasma ratios for creatinine, urine/plasma ratios for urea, urine osmolality, daily urine output, and the like (see, for example, Brenner and Lazarus (1994), in Harrison's Principles of Internal Medicine, 13th edition

Equivalents

The invention may be embodied in other specific forms without departing from the spirit or essential characteristics thereof. The foregoing embodiments are therefore to be considered in all respects illustrative rather than limiting on the invention described herein. Practice of the invention, including additional preferred aspects and embodiments thereof, will be still more fully understood from the following examples, which are presented herein for illustration only and should not be construed as limiting the invention in any way.

Examples

Example 1: Renal Failure Animal Model.

Intravenous injection of high-titer anti-rat glomerular basement membrane (GBM) serum (NTS) into WKY rats leads to deposition of rabbit IgG on rat GBM where it acts as a planted antigen and initiates a vigorous inflammatory response, characterized by up-regulation of proinflammatory cytokines and adhesion molecules such as ICAM-1 and VCAM-1, together with recruitment of leukocytes into glomeruli. The cell influx is predominantly monocytic with CD8+ cells also present, although these are not classical T cells. Some co-express CD8 and the rat monocyte/macrophage marker ED 1 (CD68 equivalent), suggesting that they represent a macrophage subset.

Cell influx is underway by day 1 of this nephrotoxic nephritis model (NTN), and glomerular leukocyte counts peak at day 3-4. Renal injury is apparent in the form of proteinuria by day 4, and fibrinoid necrosis and crescent formation occur within glomeruli by day 7.

Inflammatory interstitial infiltrates are forming by day 11, as the inflammatory process spills over from glomeruli. This "secondary" tubulointerstitial nephritis is also seen in humans with primary glomerulonephritis and often denotes a poor prognosis. Creatinine clearance (CNC) starts to fall and serum creatinine rises in the third week of NTN. Fibrosis within glomeruli and the interstitial compartment progressively worsens until death occurs around day 40 from CRF.

Briefly, Male Wistar-Kyoto (WKY) rats aged between 6 and 8 wk (weight 200-250 g) are purchased from Charles River Laboratories and fed standard rat chow and water ad libitum. Heterologous nephrotoxic serum (NTS) is prepared by standard methods. For instance, rat glomeruli are isolated, sonicated and lyophilized to provide a rat glomerular basement membrane (GMB) preparation with less than 5% tubular contamination. Albino New Zealand White rabbits are immunized with rat GBM in CFA, then boosted at monthly intervals with GBM until high titer anti-GBM antiserum is obtained at test bleeds. A total of 0.1 ml of NTS is then injected i.v. into WKY rats, leading to the development of nephrotoxic nephritis, as described above. Details of this model may be found in Tam, F.W.K. et al., Nephrology Dialysis Transplantation 14: 1658-1666 (1999).

Assessment of Renal Disease

Functional Parameters:

Albuminuria/proteinuria: this reflects glomerular leakage and, to a lesser extent, failure of tubular metabolism of filtered protein. Interpretation of such data can be difficult since it is the product of two independent variables; increased GBM permeability leads to greater proteinuria but decreased glomerular filtration rate reduces glomerular proteinuria.
Urine was collected in metabolic cages 24 h prior to harvest. Urinary albumin concentration was determined by rocket immunoelectrophoresis. Urinary protein concentration was determined by sulphosalicylic acid precipitation.
Serum creatinine and creatinine clearance (CrCl): Peripheral blood was taken at harvest for determination of serum creatinine concentration using Olympus reagents and an Olympus AU600 analyzer (Olympus, Eastleigh, U.K.). Urinary creatinine concentration was also measured (Bayer RA-XT, Newbury, U.K.) to permit calculation of creatinine clearance.
Survival: The endpoint of these studies is either sudden death or killing to relieve distress. Animals are viewed daily by a blinded, independent observer and moribund animals killed as deemed necessary by the third party. In practice, about half of animals in survival studies reached the "killing" endpoint.

Histological Parameters:

Hematoxylin and eosin stained sections allow crude assessment of glomerular scarring, tubular dropout, interstitial inflammatory infiltrates and interstitial fibrosis using arbitrary scoring scales.
Glomerular fibrosis: We estimated by computer the % renal cortical areas stained green by Masson-Trichrome histochemistry which offers a way of assessing collagen "load" within a kidney. Individual glomeruli can also be selected as the area of interest to calculate specific glomerular fibrosis. To quantify interstitial fibrosis within glomeruli, paraffin-embedded kidney sections were stained using a standard trichrome method (Martius Yellow, Brilliant Crystal Scarlet and Aniline Blue). To quantify glomerular fibrin deposition (e.g., fibrinoid necrosis), paraffin-embedded kidney sections were stained using Martius Yellow which stains fibrin a red/orange color. Sections were examined under X200 magnification using an Olympus BX40 microscope (Olympus Optical, London, U.K.) mounted with a Photonic digital camera (Photonic Science, East Sussex, U.K.). Images were captured and analyzed using Image-Pro Plus TM software (Media Cybernetics, Silver Spring, MD).
Quantitation of the % renal cortical area stained brown after immunoperoxidase staining of kidney sections for the ED(A) domain of fibronectin appears to discriminate between CRF of differing functional severity. Similarly, type III collagen immunohistochemistry allows for calculation of the % renal cortical area stained.
Glomerular alpha-smooth muscle actin (SMA) expression was measured by immunofluorescence. This protein defines a population of "myofibroblastic" cells within glomeruli, which are thought to be key players in glomerular fibrosis. Quantitation of immunofluorescent staining for alpha-SMA correlates well with glomerular Masson-trichrome fibrosis scores.

Example 2. TGFbeta Antagonist Effects in acute v. chronic stages

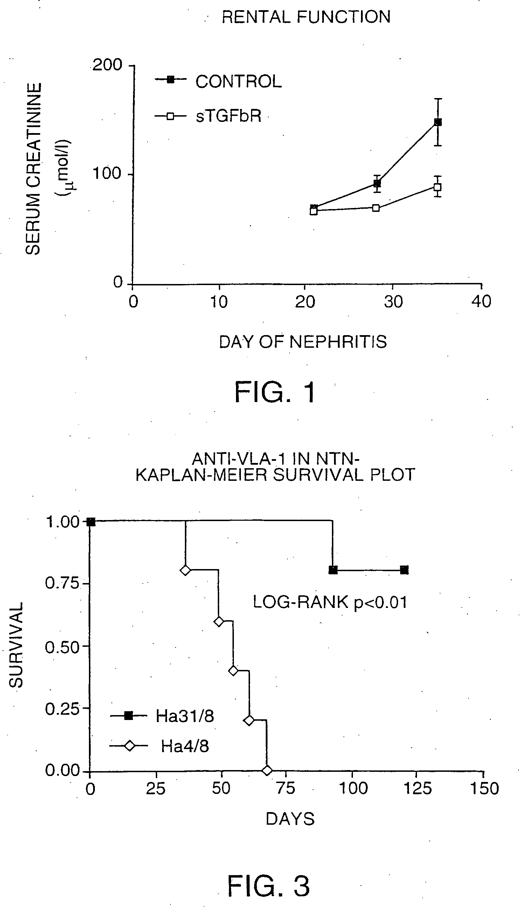

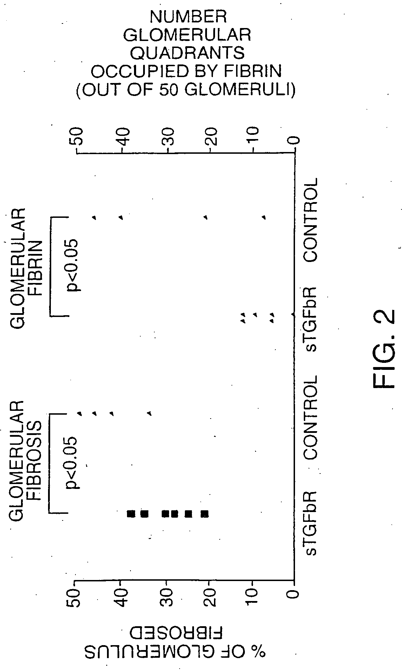

TGFbeta has a well established role in the pathogenesis of renal failure. We used a soluble dimeric rabbit TGFbeta receptor type II on a human Fc stem (see Smith, P.D., et. al., Circ. Res. 84: 1212- 1222 (1999)) to act as a TGF beta antagonist in vivo. Soluble TGFbeta receptor as an Fc fusion was given by i.p. injection every 3rd day starting on day -1 at 5 mg/kg. Administration continued for the duration of the experiment with PBS used as a control. NTN was induced as above on 12 rats on day 0 and half the rats were killed on day 9, the remained killed on day 21. No significant effects on albuminuria or creatinine clearance were seen, although there was a trend to less fibrinoid necrosis in glomeruli at all timepoints studied. No differences in crescent counts were seen between groups. Immunohistochemistry showed that the TGF beta antagonist had no significant effects on: 1) glomerular cellular infiltrates (ED1 + rriacrophages or CD8+ cells); 2) glomerular macrophage activation (inducible nitric oxide synthetase expression); or 3) glomerular cell proliferation (Ki67 expression) (Data not presented here).
In the chronic renal failure study, NTN was induced in 12 rats, and soluble dimeric rabbit TGFbeta receptor type II on a human Fc stem was given at a dose of 1 mg/kg by i.p. injection on alternate days from day 14 until day 35 when the rats were killed. Six of the twelve rats induced with NTS served as controls receiving human IgG at comparable dose. Serum creatinine was significantly lower in rats receiving the soluble TGF beta antagonist at days 28 and 35 (See Figure 1). Counting whether glomerular crescents were present or not showed no differences between groups but this was because the delay in starting treating in this experiment meant that nearly all glomeruli had some abnormalities. Shown in Figure 2 are the mean % glomerular area fibrosed (estimates for 50 consecutive glomeruli) and number of glomerular quadrants occupied by fibrin.

Example 3: Anti-VLA- 1 Antibody Homolog Effects on Acute v. Chronic Stages

Antibodies to VLA- 1 were evaluated in short term studies extending to day 10. A hamster antibody (Ha3 1/8) to the rat alpha 1 integrin VLA- 1 was administered on day -1 and then on days 1, 3, 6 and 8. A control antibody (HA4/8) was also administered. At a dosage of 2.5 mg/kg i.p., there were no significant effects on albuminuria between antagonist and control. We also saw no effects on glomerular fibrinoid necrosis or crescent formation with either antibody at any dose.

The chronic study followed a similar protocol as the soluble TGFbeta receptor study, above, except that antibodies were given from days 14-28. Twenty four rats were induced with NTS on day 0, ten received anti-VLA- 1 (Ha3 1/8) from days 14-28, ten received control antibody (Ha4/8) for the same period. Four rats were killed at day 14 for baseline histology. Ten rats were killed at day 35 for histology (5 treated and 5 control). Ten rats remained in a survival arm and either died or were killed for humane reasons. One rat in the anti-VLA- 1 mab group died from anaesthetic.

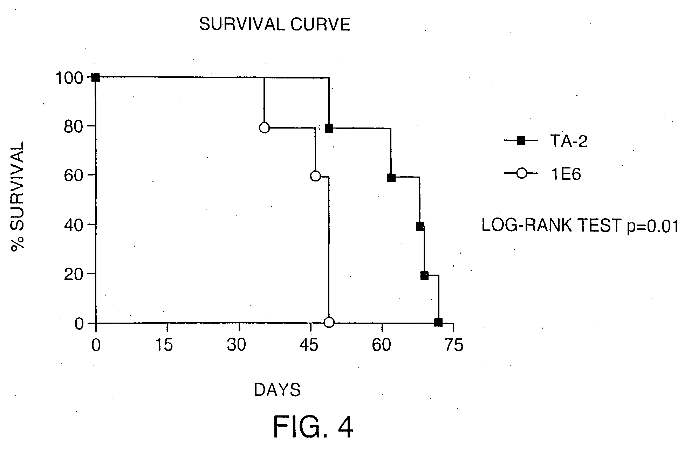

The tempo of disease in this experiment was slower than that previously seen, such that death occurred later than usual. The survival curves (Figure 3) have diverged such that all control rats have died by about 75 days and all 4 rats in the anti-VLA-1 mab group continued to look healthy even after 125 days.

Example 4: Anti-alpha 4 antibody Homolog Effects on Acute v. Chronic Stages

In the acute phase of this model, we have previously shown that alpha 4 integrin antagonists significantly attenuated renal injury (albuminuria, glomerular fibrinoid necrosis and crescent formation). See Allen, A.R. et al., J. Immunology 162: 5519-5527 (1999).

To address the role of the alpha 4 integrin antagonists in mediating chronic, progressive renal injury in this model, we used a similar protocol to that shown in Example 2 for soluble TGF beta II We used the murine antibody TA-2 (a murine IgG1 anti-rat alpha4 integrin purchased from PharMingen (San Diego)) that blocks VLA-4/alpha4beta7-mediated leukocyte adhesion. The dose used was 2.5 mg/kg, found to be effective in acute studies, and it was given i.p. on alternate days. In this study, 24 rats received NTS and 12 were given TA-2 control mab between days 5 and 13. The other 12 received TA-2 or control mab between days 13 and 21. All rats were killed at day 30. Strikingly, albuminuria was significantly reduced during the periods of TA-2 administration, although groups converged after cessation of antibody treatment. Histological injury scores suggested lower glomerular scarring in the 13-21 day TA-2 treated rats versus controls (p=0.06). There were trends in lower serum creatinine and higher CrCl in TA-2 recipients.

We followed this CRF study with a longer one, aimed at examining the survival and later histology, since CrCl curves seemed to be diverging at day 30. As for Example 2, 24 rats were induced with i.v. NTS and 4 were killed at day 14 to provide baseline histology. Anti-alpha 4 antibody TA-2 was given to ten rats between days 14 and 24, and ten rats received isotype-matched control antibody (1E6-anti-human LFA3 IgG I antibody obtained from Biogen, Inc.). Five rats from each group were killed at day 35 for histology and the remainder entered a survival study as described previously.

Survival was significantly better (median survival 68 vs. 49 days, log-rank p=0.01) in rats receiving TA-2 than controls (Figure 4). Proteinuria in TA-2 recipients was greater in day 35 onward (perhaps a marker of better preserved glomerular filtration rate) and CrCl was better preserved in these rats than in controls (p<0.05 at day 35). Interstitial scarring scores were significantly less in rats receiving anti-alpha 4 mab vs. control rats (p<0.05) and smaller differences were observed in glomerular scarring and glomerular alpha-smooth muscle actin expression, when appropriately stained sections were scored with computer image-analysis software. Using similar techniques, the extent of cortical ED(A) fibronectin deposition was also reduced in anti-alpha4 mab recipients, although no differences were seen in cortical type M collagen deposition between the two groups. Glomerular leukocyte counts were significantly greater in anti-alpha4 recipients, probably reflecting worse scarring in control rats (data not presented here).

**Claims**

1.    A method of treatment for a mammal in, or at risk of, chronic renal failure comprising administering to said mammal a therapeutically effective amount of an integrin antagonist.

2.    The method of claim 1, wherein said integrin antagonist comprises a polypeptide that antagonizes the interaction between an alpha4 subunit-containing integrin and its cognate ligand or receptor.

3.    The method of claim 2 wherein said integrin antagonist comprises an anti-VLA-4 antibody homolog.

4.    The method of claim 1, wherein said integrin antagonist comprises a polypeptide that antagonizes the interaction between an alpha1 subunit-containing integrin and its cognate ligand or receptor.

5. The method of claim 4 wherein said integrin antagonist comprises an anti-VLA-1 antibody homolog.

6. The method of claim 2, wherein the integrin antagonist is capable of antagonizing the interaction between at least one additional alpha-containing subunit of an integrin besides the alpha4 integrin subunit.

7. A method of treatment for a mammal in, or at risk of, chronic renal failure comprising administering to said mammal a therapeutically effective amount of two or more integrin antagonists, wherein at least two of the antagonists include a first integrin antagonist that antagonizes the interaction between an alpha4-containing subunit and its cognate ligand or receptor, and a second integrin antagonist that antagonizes the interaction between another alpha-containing subunit that is not alpha4.

8. The method of claims 1 or 6, wherein the antagonist is a small molecule.

9. A method as claims 1 or 7, wherein said mammal is afflicted with a condition selected from the group consisting of chronic renal failure, end-stage renal disease, chronic diabetic nephropathy, diabetic glomerulopathy, diabetic renal hypertrophy, hypertensive nephrosclerosis, hypertensive glomerulosclerosis, chronic glomerulonephritis, hereditary nephritis, and renal dysplasia.

10. The method of claim 2, wherein the antibody homolog is a humanized antibody having a portion thereof encoded by a nucleic acid sequence comprising a nucleic acid that hybridizes under high stringency conditions to a nucleic acid sequence selected from the group of sequences in Table 6 of U. S. Patent 5,840,299 or the complement of said nucleic acid sequences.

11. The method of claim 2, wherein the antibody homolog is a humanized antibody having a portion thereof encoded by a nucleic acid sequence comprising a nucleic acid that hybridizes under low stringency conditions to a nucleic acid sequence selected from the group of nucleic acid sequences in Table 6 of U. S. Patent 5,840,299, or the complement of said nucleic acid sequences.

12. The method of claim 2, wherein the antibody homolog is a humanized antibody having a portion thereof encoded by a nucleic acid sequence comprising a nucleic acid that hybridizes under low stringency conditions to a nucleic acid sequence encoding a polypeptide sequence selected from the group consisting of: a) SEQ NO: 2 found in U. S. Patent 5,932,214; b) SEQ ID NO: 4 found in U. S. Patent 5,932,214; and c) a variable domain of the antibody produced by cell line ATCC

13. The method of claim 2, wherein the antibody homolog is a humanized antibody having a portion thereof encoded by a nucleic acid sequence comprising a nucleic acid that hybridizes under high stringency conditions to a nucleic acid sequence encoding a polypeptide sequence selected from the group consisting of: a) SEQ ID NO: 2 found in U. S. Patent 5,932,214; b) SEQ ID NO: 4 found in U. S. Patent 5,932,214; and c) a variable domain of the antibody produced by cell line ATCC CRL 11175.

14. The method of claim 7, wherein the first integrin antagonist is a humanized antibody having a portion thereof encoded by a nucleic acid sequence comprising a nucleic acid that hybridizes under high stringency conditions to a nucleic acid sequence selected from the group of sequences in Table 6 of U. S. Patent 5,840,299 or the complement of said nucleic acid sequences.

15. The method of claim 7, wherein the first integrin antagonist is a humanized antibody having a portion thereof encoded by a nucleic acid sequence comprising a nucleic acid that hybridizes under low stringency conditions to a nucleic acid sequence selected from the group of nucleic acid sequences in Table 6 of U. S. Patent 5,840,299 or the complement of said nucleic acid sequences.

16. The method of claim 7, wherein the first integrin antagonist is a humanized antibody having a portion thereof encoded by a nucleic acid sequence comprising a nucleic acid that hybridizes under low stringency conditions to a nucleic acid sequence encoding a polypeptide sequence selected from the group consisting of: a) SEQ ID NO: 2 found in U. S. Patent 5,932,214; b) SEQ ID NO: 4 found in U. S. Patent 5,932,214; and c) a variable domain of the antibody produced by cell line ATCC CRL 11175.

17. The method of claim 7, wherein the first integrin antagonist is a humanized antibody having a portion thereof encoded by a nucleic acid sequence comprising a nucleic acid that hybridizes under high stringency conditions to a nucleic acid sequence encoding a polypeptide sequence selected from the group consisting of: a) SEQ ID NO: 2 found in U. S. Patent 5,932,214; b) SEQ ID NO: 4 found in U. S. Patent 5,932,214; and c) a variable domain of the antibody produced by cell line ATCC CRL 11175.

**18.** A method as claimed in claim 1 or 7, wherein said mammal is afflicted with chronic rejection following renal allograft transplantation.

**19.** The method of claims 3 or 5, wherein the antibody homolog is a Fab fragment, Fab' fragment, F(ab')2 fragment, or F(v) fragment.

**20.** The method of claims 3 or 5, wherein the antibody homolog is an IgG.

**21.** The method of claim 1, wherein the mammal has a measured glomerular filtration rate (GFR) chronically less than about 50% of the expected GFR.

**22.** The method of claim 1, wherein the mammal is a human subject or patient.

**23.** The method of claim 1, wherein treatment inhibits, delays or alleviates the progressive loss of renal function.

RENTAL FUNCTION

**FIG. 1**

ANTI-VLA-1 IN NTN-
KAPLAN-MEIER SURVIVAL PLOT

**FIG. 3**

FIG. 2

SURVIVAL CURVE

LOG-RANK TEST p=0.01

FIG. 4

EP 1 700 606 A1

**European Patent Office**

# EUROPEAN SEARCH REPORT

Application Number

EP 06 00 9570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | A. MOLINA ET AL.: "Prevention of mercuric chloride-induced nephritis in the Brown Norway rat by treatment with antibodies against the alpha4 integrin."<br>THE JOURNAL OF IMMUNOLOGY,<br>vol. 153, no. 5,<br>1 September 1994 (1994-09-01), pages 2313-2320, XP002152504<br>Baltimore, MD, USA<br>* the whole document * | 1-3,8,9 | INV.<br>A61K39/395<br>A61P13/12<br><br>ADD.<br>A61K38/17<br>C12N15/13 |
| Y | | 10-13 | |
| X | WO 98/04247 A (BIOGEN, INC.)<br>5 February 1998 (1998-02-05)<br>* page 3, line 4 - line 12 *<br>* page 7, line 19 - line 20 * | 1-3,8,9 | |
| X | F. TAYLOR ET AL.: "7E3 F(ab')2, a monoclonal antibody to the platelet GPIIb/IIIa receptor, protects against microangiopathic hemolytic anemia and microvascular thrombotic renal failure in baboons treated with C4b binding protein and a sublethal infusion of Escherichia coli."<br>BLOOD,<br>vol. 89, no. 11, 1 June 1997 (1997-06-01), pages 4078-4084, XP000960528<br>New York, NY, USA<br>* abstract *<br>* figure 3 * | 1,8,9 | TECHNICAL FIELDS SEARCHED (IPC)<br><br>C07K |

-/--

The present search report has been drawn up for all claims

| Place of search | Date of completion of the search | Examiner |
|---|---|---|
| Munich | 21 July 2006 | Luzzatto, E |

EPO FORM 1503 03.82 (P04C01)

European Patent
Office

**EUROPEAN SEARCH REPORT**

Application Number

EP 06 00 9570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | H. RABB ET AL.: "The leukointegrin alpha d/beta2 (alphad/CD18): specific changes in surface expression in patients on hemodialysis" CELL ADHESION AND COMMUNICATION, vol. 6, no. 1, June 1998 (1998-06), pages 13-20, XP000960574 * abstract * * page 19, right-hand column, line 10 - line 28 * | 1,8,9 | |
| D,Y | US 5 840 299 A (BENDIG ET AL.) 24 November 1998 (1998-11-24) * examples * * claims * * table 6 * | 10,11 | |
| A | | 14,15 | |
| D,Y | US 5 932 214 A (LOBB ET AL.) 3 August 1999 (1999-08-03) * examples II-IV * * examples * seq. id. nos. 2 and 4 | 12,13 | |
| A | | 16,17 | TECHNICAL FIELDS SEARCHED (IPC) |
| A | K. LIN ET AL.: "Very late antigen (VLA4) antagonists as anti-inflammatory agents." CURRENT OPINION IN CHEMICAL BIOLOGY, vol. 2, no. 4, August 1998 (1998-08), pages 453-457, XP000869619 London, GB * the whole document * | 1-3,8, 10-13 | |

-/--

The present search report has been drawn up for all claims

| Place of search | Date of completion of the search | Examiner |
|---|---|---|
| Munich | 21 July 2006 | Luzzatto, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

**EUROPEAN SEARCH REPORT**

Application Number

EP 06 00 9570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | N. BRAUN: "Expression of adhesion molecules and activation markers on lymphocytes and monocytes during hemodialysis." BLOOD PURIFICATION, vol. 15, no. 2, March 1997 (1997-03), pages 61-76, XP000960572 Basel, Switzerland * abstract * * tables 1-3 * * page 73, left-hand column, line 3 - page 74, left-hand column, line 12 * | 1-17 | |
| A | VON WILLEBRAND E ET AL: "Adhesion molecules and their ligands in chronic rejection of human renal allografts." TRANSPLANTATION PROCEEDINGS. 1997 FEB-MAR, vol. 29, no. 1-2, February 1997 (1997-02), pages 1530-1531, XP002391504 ISSN: 0041-1345 * the whole document * | 1,18-23 | |
| A | ROSSMANN P ET AL: "[Immunohistochemistry of a biopsy of an allotransplanted kidney]" CASOPIS LÉKAR C ESKYCH. 14 DEC 1998, vol. 137, no. 24, 14 December 1998 (1998-12-14), pages 757-762, XP002391505 ISSN: 0008-7335 * the whole document * | 1,18-23 | |

TECHNICAL FIELDS SEARCHED (IPC)

-/--

The present search report has been drawn up for all claims

| Place of search | Date of completion of the search | Examiner |
|---|---|---|
| Munich | 21 July 2006 | Luzzatto, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 06 00 9570

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KORCZAK-KOWALSKA G ET AL: "Lymphocyte adhesion molecules in renal allograft rejection." TRANSPLANTATION PROCEEDINGS. DEC 1996, vol. 28, no. 6, December 1996 (1996-12), pages 3437-3438, XP009069807 ISSN: 0041-1345 * the whole document * | 1,18-23 | |

TECHNICAL FIELDS
SEARCHED (IPC)

The present search report has been drawn up for all claims

| Place of search | Date of completion of the search | Examiner |
|---|---|---|
| Munich | 21 July 2006 | Luzzatto, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                                    
& : member of the same patent family, corresponding document

EPO FORM 1503 03.82 (P04C01)

European Patent
Office

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

European Patent Office

**LACK OF UNITY OF INVENTION
SHEET B**

Application Number

EP 06 00 9570

The Search Division considers that the present European patent application does not comply with the requirements of unity of invention and relates to several inventions or groups of inventions, namely:

1. claims: 1 (partially), 2, 3, 6 and 7 (all completely), 8 and 9 (all partially), 10-17 (all completely), 18-23 (all partially)

   Method of treatment for a mammal in, or at risk of, chronic renal failure comprising administering to said mammal a therapeutically effective amount of an integrin antagonist, wherein said integrin antagonist comprises a polypeptide that antagonizes the interaction between an alpha4 subunit-containing integrin and its cognate ligand or receptor. Said method may additionally comprise a second integrin antagonist that antagonizes the interaction between another alpha-containing subunit that is not alpha4.

   ---

2. claims: 1 (partially), 4 and 5 (both completely), 8 and 9 (both partially), 18-23 (all partially)

   Method of treatment for a mammal in, or at risk of, chronic renal failure comprising administering to said mammal a therapeutically effective amount of an integrin antagonist, wherein said integrin antagonist comprises a polypeptide that antagonizes the interaction between an alpha1 subunit-containing integrin and its cognate ligand or receptor.

   ---

## ANNEX TO THE EUROPEAN SEARCH REPORT
## ON EUROPEAN PATENT APPLICATION NO.

EP 06 00 9570

This annex lists the patent family members relating to the patent documents cited in the above-mentioned European search report.
The members are as contained in the European Patent Office EDP file on
The European Patent Office is in no way liable for these particulars which are merely given for the purpose of information.

21-07-2006

| Patent document cited in search report | | Publication date | Patent family member(s) | | Publication date |
|---|---|---|---|---|---|
| WO 9804247 | A | 05-02-1998 | AU | 3738597 A | 20-02-1998 |
| | | | AU | 737372 B2 | 16-08-2001 |
| | | | AU | 3738697 A | 20-02-1998 |
| | | | BG | 64470 B1 | 30-04-2005 |
| | | | BG | 103193 A | 30-09-1999 |
| | | | BG | 108806 A | 30-04-2005 |
| | | | BR | 9710570 A | 28-06-2005 |
| | | | CA | 2261848 A1 | 05-02-1998 |
| | | | CN | 1478472 A | 03-03-2004 |
| | | | CN | 1230110 A | 29-09-1999 |
| | | | CZ | 9900232 A3 | 16-06-1999 |
| | | | EA | 5526 B1 | 28-04-2005 |
| | | | EE | 9900032 A | 16-08-1999 |
| | | | EP | 0914605 A1 | 12-05-1999 |
| | | | EP | 0917462 A1 | 26-05-1999 |
| | | | IL | 128221 A | 10-12-2003 |
| | | | JP | 2000516596 T | 12-12-2000 |
| | | | KR | 2000029538 A | 25-05-2000 |
| | | | NO | 990338 A | 25-03-1999 |
| | | | NZ | 333904 A | 23-06-2000 |
| | | | SK | 8199 A3 | 10-04-2000 |
| | | | TR | 9900781 T2 | 21-07-1999 |
| | | | WO | 9804913 A1 | 05-02-1998 |
| US 5840299 | A | 24-11-1998 | AU | 703152 B2 | 18-03-1999 |
| | | | AU | 1696095 A | 08-08-1995 |
| | | | CA | 2182013 A1 | 27-07-1995 |
| | | | CN | 1140413 A | 15-01-1997 |
| | | | EP | 0804237 A1 | 05-11-1997 |
| | | | FI | 962958 A | 24-09-1996 |
| | | | HU | 75129 A2 | 28-04-1997 |
| | | | JP | 9508272 T | 26-08-1997 |
| | | | JP | 2006045237 A | 16-02-2006 |
| | | | NZ | 279730 A | 27-04-1998 |
| | | | PL | 315634 A1 | 25-11-1996 |
| | | | WO | 9519790 A1 | 27-07-1995 |
| US 5932214 | A | 03-08-1999 | NONE | | |

EPO FORM P0459

For more details about this annex : see Official Journal of the European Patent Office, No. 12/82

## REFERENCES CITED IN THE DESCRIPTION

**Patent documents cited in the description**

- US 4833092 A, Geysen
- US 9104862 W
- US 9713013 B
- WO 9415958 A
- WO 9200995 A
- WO 9308823 A
- WO 9208464 A
- US 5260277 A
- US 5510332 A
- WO 9853814 A
- WO 9703094 A
- WO 9702289 A
- WO 9640781 A
- WO 9622966 A
- WO 9620216 A
- WO 9601644 A
- WO 96106108 A
- WO 9515973 A
- WO 9309229 A
- US 5411941 A
- US 5266683 A
- US 5011691 A
- US 5855888 A
- US 5798230 A
- US 5789650 A
- EP 0239400 A, Winter
- WO 9007861 A
- US 5693762 A
- US 00479893 A
- US 9400266 B
- US 9501219 W
- US 5840299 A
- US 5932214 A
- US 5100646 A
- US 5335660 A

**Non-patent literature cited in the description**

- **ALLEN et al.** *Journal of Immunology,* 1999, vol. 162, 5519-5527
- **TAM, F.W.K. et al.** *Nephrol. Dial. Transplant.,* 1999, vol. 14, 1658-1666
- **E. C. BUTCHER.** *Cell,* 1991, vol. 67, 1033
- **D. COX et al.** The Pharmacology of the Integrins. *Medicinal Research Rev,* 1994, vol. 195
- Cell Adhesion Integrins as Pharmaceutical Targets. **V. W. ENGLEMAN et al.** Ann. Rev. Medicinal Chemistry. Acad. Press, 1996, vol. 31, 191
- **GOTWALS et al.** *J. Clin. Invest.,* 1996, vol. 97, 2469-2477
- **R. LOBB ; M. HEMLER.** *J. Clin: Invest.,* 1994, vol. 94, 1722-1728
- **DAYHOFF et al.** Atlas of Protein Sequence and Structure. Nat. Biomed. Res. Foundation, 1978, vol. 5, 354-352
- **KARLIN ; ALTSCHUL.** *Proc. Nat. Acad. Sci., USA,* 1990, vol. 87, 2264
- **KARLIN ; ALTSCHUL.** *Proc. Nat. Acad. Sci., USA,* 1993, vol. 90, 5873
- **ALTSCHUL et al.** *J. Mol. Biol.,* 1990, vol. 215, 403
- **ALTSCHUL.** *Nucleic Acids Res.,* 1997, vol. 25, 3389
- Current Protocols in Molecular Biology. John Wiley & Sons, Inc, 1989
- **BRENNER ; LAZARUS et al.** Harrison's Principles of Internal Medicine. McGraw Hill, 1994
- **LUKE ; STROM.** Internal Medicine. Mosby-Year Book, Inc, 1994
- **LEMANN et al.** *Am. J. Kidney-Dis.,* 1990, vol. 16 (3), 236-243
- **OSBORN et al.** *Cell,* 1989, vol. 59, 1203-1211
- **J.J. DEVLIN et al.** *Science,* 1990, vol. 249, 400-406
- **J.K. SCOTT ; G.P. SMITH.** *Science,* 1990, vol. 249, 386-390
- **KOMORIYA et al.** The Minimal Essential Sequence for a Major Cell Type-Specific Adhesion Site (CS1) Within the Alternatively Spliced Type III Connecting Segment Domain of Fibronectin Is Leucine-Aspartic Acid-Valine. *J. Biol. Chem.,* 1991, vol. 266 (23), 15075-79
- **T. A. FERGUSON et al.** Two Integrin Binding Peptides Abrogate T-cell Mediated Immune Responses In Vivo. *Proc. Natl. Acad. Sci. USA,* 1991, vol. 88, 8072-76
- **S. M. WAHL et al.** Synthetic Fibronectin Peptides Suppress Arthritis in Rats by Interrupting Leukocyte Adhesion and Recruitment. *J. Clin. Invest.,* 1994, vol. 94, 655-62

- **D.M. NOWLIN et al.** A Novel Cyclic Pentapeptide Inhibits Alpha4Betal Integrin-mediated Cell Adhesion. *J. Biol. Chem.,* 1993, vol. 268 (27), 20352-59
- **D. Y. JACKSON et al.** Potent a4betal peptide antagonists as potential anti-inflammatory agents. *J. Med. Chem.,* 1997, vol. 40, 3359
- **H. SHROFF et al.** Small peptide Inhibitors of alpha4beta7 mediated MadCAM-1 adhesion to lymphocytes. *Bio. Med, Chem. Lett.,* 1996, vol. 1, 2495
- **SCOTT ; SMITH.** Searching for Peptide Ligands with an Epitope Library. *Science,* 1990, vol. 249, 386-90
- **DEVLIN et al.** Random Peptide Libraries: A Source of Specific Protein Binding Molecules. *Science,* 1990, vol. 249, 40407
- **MENDRICK et al.** *Lab. Invest.,* 1995, vol. 72, 367-375
- **SONNENBERG et al.** *J. Biol. Chem.,* 1987, vol. 262, 10376-10383
- **YAO et al.** *J Cell Sci,* 1996, vol. 109, 3139-50
- **HEMLER et al.** *J Immunol,* 1984, vol. 132, 3011-8
- **PISCHEL et al.** *J Immunol,* 1987, vol. 138, 226-33
- **WAYNER et al.** *J Cell Biol,* 1988, vol. 107, 1881-91
- **HEMLER et al.** *J Biol Chem,* 1987, vol. 262, 11478-85
- **WANG et al.** *Am, J. Respir. Cell Mol. Biol.,* 1996, vol. 15, 664-672
- **DAVIES et al.** *J Cell Biol,* 1989, vol. 109, 1817-26
- **SANCHEZ-MADRID et al.** *Proc Natl Acad Sci U S A,* 1982, vol. 79, 7489-93
- **DIAMOND et al.** *J Cell Biol,* 1993, vol. 120, 1031-43
- **STACKER et al.** *J Immunol,* 1991, vol. 146, 648-55
- **VAN DER VIEREN et al.** *Immunity,* 1995, vol. 3, 683-90
- **BENNETT et al.** *Proc Natl Acad Sci U S A,* 1983, vol. 80, 2417-21
- **HESSLE et al.** *Differentiation,* 1984, vol. 26, 49-54
- **WEINACKER et al.** *J Biol Chem,* 1994, vol. 269, 6940-8
- **CERF-BENSUSSAN et al.** *Eur J Immunol,* 1992, vol. 22, 273-7
- **NISHIMURA et al.** *J Biol Chem,* 1994, vol. 269, 28708-15
- **BOSSY et al.** *EMBO J,* 1991, vol. 10, 2375-85
- **CAMPER et al.** *J. Biol. Chem.,* 1998, vol. 273, 20383-20389
- **KOHLER.** *Nature,* 1975, vol. 265, 295-297
- **SANCHEZ-MADRID et al.** *Eur. ,J. Immunol.,* 1986, vol. 16, 1343-1349
- **HEMLER et al.** *J. .Biol. Chem.,* 1987, vol. 262, 11478-11485
- **ELICES et al.** *Cell,* 1990, vol. 60, 577-584
- **PULIDO et al.** *J. Biol. Chem.,* 1991, vol. 266 (16), 10241-10245
- **BOEMER et al.** *J. Immunol.,* 1991, vol. 147, 86-95
- **PERSSON et al.** *Proc. Nat. Acad Sci.USA,* 1991, vol. 88, 2432-2436
- **HUANG ; STOLLAR.** *J. Immunol. Methods,* 1991, vol. 141, 227-236
- **RIECHMANN et al.** *Nature,* 1988, vol. 332, 323-327
- **VERHOEYEN et al.** *Science,* 1988, vol. 239, 1534-1536
- **JONES et al.** *Nature,* 1986, vol. 321, 522-525
- **RIECHMANN.** *Nature,* 1988, vol. 332, 323-327
- **QUEEN et al.** *Proc. Nat. Acad. Sci. USA,* 1989, vol. 86, 10029
- **ORLANDI.** *Proc. Nat. Acad. Sci. USA,* 1989, vol. 86, 3833
- **TEMPEST et al.** *Biotechnology,* 1991, vol. 9, 266-271
- **LEGER, O.J. et al.** *Hum. Antibodies,* 1997, vol. 8, 3-16
- Comprehensive Medicinal Chemistry. Pergamon Press, 1990, 970-986
- Remington's Pharmaceutical Sciences. Mack Pub, 1990
- **BRENNER ; LAZARUS.** Harrison's Principles of Internal Medicine. 1994
- **TAM, F.W.K. et al.** *Nephrology Dialysis Transplantation,* 1999, vol. 14, 1658-1666
- **SMITH, P.D.** *Circ. Res.,* 1999, vol. 84, 1212-1222
- **ALLEN, A.R. et al.** *J. Immunology,* 1999, vol. 162, 5519-5527